# FASCICULE DE BREVET EUROPEEN

(11) **EP 3 570 978 B1**
(45) Date de publication et mention de la délivrance du brevet: **11.08.2021**
(21) Numéro de dépôt: 18700429.6
(22) Date de dépôt: 18.01.2018
(51) Int. Cl.: B01L 3/00, C12N 5/071, G01N 33/50

(54) **DISPOSTIF ET PROCEDE DE DISCRIMINATION DE SPERMATOZOÏDES**
VORRICHTUNG UND VERFAHREN ZUR UNTERSCHEIDUNG VON SPERMATOZOEN
DEVICE AND METHOD FOR DISCRIMINATING SPERMATOZOA

(30) Priorité: 18.01.2017 FR 1750368
(43) Date de publication de la demande: 27.11.2019
(73) Titulaire: Genes Diffusion, 59500 Douai (FR)
(72) Inventeur: TREIZEBRE, Anthony, 59310 Coutiches (FR); THOMMEN, Quentin, 59260 Lille-Hellemmes (FR); PESEZ, Jean, 59130 Lambersart (FR); DAMART, Hervé, 59118 Wambrechies (FR); LIEGEOIS, Luc, 59283 Raimbeaucourt (FR); COURTADE, Emmanuel, 59260 Lille-Hellemmes (FR)
(74) Mandataire: Matkowska, Franck
(86) Numéro de dépôt international: PCT/EP2018/051154
(87) Numéro de publication internationale: WO 2018/134281

(56) Documents cités:
- FR-A1- 3 024 738
- US-A1- 2014 273 059
- US-A1- 2016 054 222
- JOHNSON L A: "Sexing mammalian sperm for production of offspring: The state-of-the-art", ANIMAL REPRODUCTION SCIENCE, ELSEVIER SCIENCE PUBLISHERS, AMSTERDAM, NL, vol. 60-61, no. Special Issue, 2 juillet 2000 (2000-07-02), pages 93-107, XP002567974, ISSN: 0378-4320, DOI: 10.1016/S0378-4320(00)00088-9

## Description

### Domaine technique

La présente invention concerne le domaine de la discrimination *in vitro* de spermatozoïdes en fonction de leur chromosome X ou Y, notamment de spermatozoïdes, marqués au moyen d'au moins un fluorochrome.

### Art antérieur

Un spermatozoïde est une cellule de type haploïde, qui ne contient généralement qu'un seul exemplaire de chaque chromosome, par exemple un chromosome X ou Y, et dont la motilité est assurée par un flagelle.

Dans le domaine de la reproduction animale, on a développé à ce jour différentes solutions de sexage, qui permettent de discriminer et trier automatiquement des spermatozoïdes en fonction de leur chromosome X ou Y, et qui sont basées sur la cytométrie de flux.

D'une manière générale, toutes ces solutions techniques de sexage par cytométrie de flux reposent sur le fait que les spermatozoïdes X contiennent environ 4% d'ADN de plus que les spermatozoïdes Y, notamment dans l'espèce bovine. Ainsi, dans ces solutions techniques de sexage :
(a) on marque les spermatozoïdes avec un intercalant d'ADN fluorescent, généralement désigné fluorochrome ; les spermatozoïdes X, qui sont présumés contenir une plus grande quantité d'ADN absorbent une quantité plus importante de fluorochrome que les spermatozoïdes Y ;
(b) on injecte une solution contenant les spermatozoïdes marqués dans un canal de transport, et le cas échéant on les soumet à une focalisation hydrodynamique de manière à les aligner et les faire circuler dans ce canal de transport,
(c) on excite les spermatozoïdes marqués avec un rayonnement approprié, afin qu'ils émettent un rayonnement lumineux par fluorescence,
(d) on discrimine les spermatozoïdes en fonction de chromosome X ou Y en détectant l'intensité du rayonnement lumineux émis par fluorescence (intensité de fluorescence), et
(e) on trie automatiquement chaque spermatozoïde X ou Y en fonction de cette intensité de fluorescence détectée.

Une première méthode de tri connue (étape (e) susvisée) consiste à soumettre la solution contenant les spermatozoïdes marqués à des vibrations au moyen d'un transducteur piézoélectrique, de manière à former des microgouttelettes, qui dans la mesure du possible ne contiennent chacune qu'un seul spermatozoïde, puis à charger électriquement chaque gouttelette en fonction de l'intensité de fluorescente détectée, et enfin à faire passer successivement les microgouttelettes chargées électriquement dans un champ électrique permettant de séparer les gouttelettes chargées contenant un spermatozoïde X des gouttelettes chargées contenant un spermatozoïde Y. Cette méthode de tri est décrite par exemple dans les demandes de brevet internationales suivantes : WO2004/104178, WO2004/017041, WO2009/014643, WO2009/151624.

On a proposé dans la demande de brevet internationale WO2010/001254 une autre solution dans laquelle le tri des spermatozoïdes est effectué au moyen d'un rayonnement électromagnétique, de type faisceau laser, choisi de manière à altérer de manière sélective, en fonction de l'intensité de fluorescence détectée, les spermatozoïdes marqués et transportés l'un derrière l'autre dans un canal de transport.

Des études ont par ailleurs montré que l'intensité maximale du rayonnement lumineux émis par fluorescence par les spermatozoïdes marqués était un paramètre qui pouvait, dans une certaine mesure, être utilisé pour discriminer des spermatozoïdes en fonction de chromosome X ou Y. Néanmoins, en pratique il s'avère que ce paramètre n'est pas très fiable car la discrimination dépend fortement notamment de la qualité de la semence et du protocole de marquage. En outre le rendement de la détection peut être faible car seule une fraction des spermatozoïdes peut en pratique être discriminée avec une fiabilité.

Dans la demande de brevet américain US2010/0167336, on a récemment proposé une méthode de discrimination des spermatozoïdes X/Y basée sur une mesure de la longueur du noyau des spermatozoïdes. Dans cette publication, les spermatozoïdes marqués sont transportés dans un canal de transport à section circulaire et on mesure la longueur du noyau de chaque spermatozoïde lors de son passage dans un faisceau laser. Il semblerait toutefois que la fiabilité des résultats de cette méthode et leur reproductibilité ne soient pas avérées. Dans la demande de brevet américain US2014/273059 on a proposé la détermination de l'orientation spatiale des spermatozoïdes dans un canal de circulation au moyen de la durée d'une impulsion de fluorescence.

Il existe donc à ce jour un besoin de trouver une solution technique de discrimination de spermatozoïdes de mammifères, notamment de spermatozoïdes d'animaux, qui soit efficace, et qui pallie les inconvénients susvisés des solutions de l'art antérieur.

### Objectif de l'invention

La présente invention a pour objectif de proposer une nouvelle solution technique de discrimination de spermatozoïdes marqués au moyen d'au moins un fluorochrome, en fonction de leur chromosome X ou Y, qui pallie notamment les inconvénients susvisés des solutions de l'art antérieur.

### Résumé de l'invention

Selon un premier aspect, l'invention a pour objet un dispositif permettant de discriminer des spermatozoïdes marqués au moyen d'au moins un fluorochrome, lequel dispositif comporte un canal de transport, dans lequel peut circuler une solution contenant lesdits spermatozoïdes marqués dans une direction de transport (Y) prédéfinie, des moyens d'excitation permettant d'exciter l'émission par fluorescence des spermatozoïdes marqués circulant dans ledit canal de transport, des moyens de détection de la fluorescence émise par les spermatozoïdes marqués circulant dans ledit canal de transport, et des moyens électroniques de traitement permettant de discriminer les spermatozoïdes marqués circulant dans le canal de transport à partir de la fluorescence détectée par les moyens de détection de la fluorescence : ledit canal de transport comporte un plan de symétrie principal (P1) parallèle à la direction de transport (Y), et une section transversale, dans un plan (X,Z) perpendiculaire à la direction de transport (Y), qui est symétrique, qui est caractérisée par un axe de symétrie principal (A) et présente une dimension transversale maximale (H), mesurée le long de cet axe de symétrie principal (A), et une dimension transversale minimale (E), inférieure à la dimension transversale maximale (H), et mesurée le long d'un axe transverse secondaire (B) perpendiculaire à l'axe de symétrie principal (A) ; les moyens de détection de fluorescence comportent au moins des premiers moyens de détection, qui sont aptes à détecter la fluorescence émise par les spermatozoïdes dans une direction essentiellement perpendiculaire au plan de symétrie principal (P1) du canal de transport, et plus particulièrement au moins dans une direction perpendiculaire au plan de symétrie principal (P1) du canal de transport, et les moyens électroniques de traitement sont aptes à discriminer automatiquement les spermatozoïdes en utilisant au moins la durée de chaque signature de fluorescence caractéristique d'un spermatozoïde dans le signal de détection de fluorescence généré par les premiers moyens de détection.

Selon un deuxième aspect, l'invention a également pour objet un dispositif permettant de discriminer des spermatozoïdes marqués au moyen d'au moins un fluorochrome, lequel dispositif comporte un canal de transport, dans lequel peut circuler une solution contenant lesdits spermatozoïdes marqués dans une direction de transport (Y) prédéfinie, des moyens d'excitation permettant d'exciter l'émission par fluorescence des spermatozoïdes marqués circulant dans ledit canal de transport, des moyens de détection de la fluorescence émise par les spermatozoïdes marqués circulant dans ledit canal de transport, et des moyens électroniques de traitement permettant de discriminer les spermatozoïdes marqués circulant dans le canal de transport à partir de la fluorescence détectée par les moyens de détection de la fluorescence ; ledit dispositif comporte des moyens permettant le transport des spermatozoïdes l'un derrière l'autre dans le canal de transport avec sensiblement la même orientation spatiale de chaque spermatozoïde ; les moyens de détection de fluorescence comportent au moins des premiers moyens de qui sont orientés en direction de l'une des deux faces principales de plus grande surface de chaque spermatozoïde passant successivement dans le canal de transport devant lesdits moyens de détection en étant orienté spatialement ; les moyens électroniques de traitement sont aptes à discriminer automatiquement les spermatozoïdes en utilisant au moins la durée de chaque signature de fluorescence caractéristique d'un spermatozoïde dans le signal de détection de fluorescence délivré par les premiers moyens de détection.

Plus particulièrement, selon ledit premier ou deuxième aspect, le dispositif de l'invention peut comporter les caractéristiques additionnelles et facultatives ci-après, prises isolément ou en combinaison les unes avec les autres et définies dans l'une quelconque des revendications 2 à 12.

L'invention a également pour objet une utilisation du dispositif susvisé pour discriminer automatiquement, et le cas échéant sélectionner automatiquement ou trier automatiquement, *in vitro* des spermatozoïdes.

L'invention a également pour objet un procédé de discrimination *in vitro* de spermatozoïdes au cours duquel on injecte, dans le canal de transport du dispositif susvisé une solution contenant les spermatozoïdes marqués au moyen d'au moins un fluorochrome, on fait circuler dans le canal de transport lesdits spermatozoïdes les uns derrière les autres en les orientant spatialement avec la même orientation spatiale, et on détecte automatiquement le type de chromosome de chaque spermatozoïde circulant dans le canal de transport, en utilisant au moins la durée (D) de la signature de fluorescence caractéristique d'un spermatozoïde dans le signal de détection de fluorescence délivré par les premiers moyens de détection du dispositif.

L'invention a également pour objet un procédé production d'une semence sexée par sélection ou tri *in vitro* de spermatozoïdes au moyen du dispositif susvisé, au cours duquel on injecte, dans le canal de transport du dispositif une solution contenant les spermatozoïdes marqués au moyen d'au moins un fluorochrome, on fait circuler dans le canal de transport lesdits spermatozoïdes les uns derrière les autres en les orientant spatialement avec la même orientation spatiale, on détecte automatiquement le type de chromosome de chaque spermatozoïde circulant dans le canal de transport, et notamment si le spermatozoïde est de type X ou Y, en utilisant au moins la durée (D) de chaque signature de fluorescence caractéristique d'un spermatozoïde dans le signal de détection de fluorescence délivré par les premiers moyens de détection du dispositif, et on sélectionne les spermatozoïdes d'un type donné, notamment en altérant les spermatozoïdes de l'autre type, ou on trie les spermatozoïdes en fonction de leur type, et notamment de leur type X ou Y, notamment séparant les spermatozoïdes d'un type donné des spermatozoïdes de l'autre type.

### Brève description des figures

Les caractéristiques et avantages de l'invention apparaîtront plus clairement à la lecture de la description détaillée ci-après de plusieurs variantes de réalisation de l'invention, laquelle description est donnée à titre d'exemple non limitatif et non exhaustif de l'invention, et en référence aux dessins annexés sur lesquels :
- la figure 1 est une représentation schématique d'une première variante de réalisation d'un dispositif de sélection de l'invention, la puce microfluidique de ce dispositif de sélection étant représentée en vue de dessus ;
- la figure 2 est une vue en coupe transversale de la puce microfluidique de ce dispositif de sélection, dans le plan de coupe II-II de la figure 1 ;
- la figure 3 est une vue en coupe transversale de ce dispositif de tri, dans le plan de coupe III-III de la figure 1 ;
- la figure 4 est une vue en coupe transversale de ce dispositif de tri, dans le plan de coupe IV-IV de la figure 3 ;
- la figure 5 est une vue en coupe transversale de ce dispositif de tri, dans le plan de coupe V-Vde la figure 1 ;
- la figure 6 est une vue en coupe transversale de ce dispositif de tri, dans le plan de coupe VI-VI de la figure 1 ;
- la figure 7 est une vue en coupe transversale de ce dispositif de tri, dans le plan de coupe VII-VII de la figure 1;

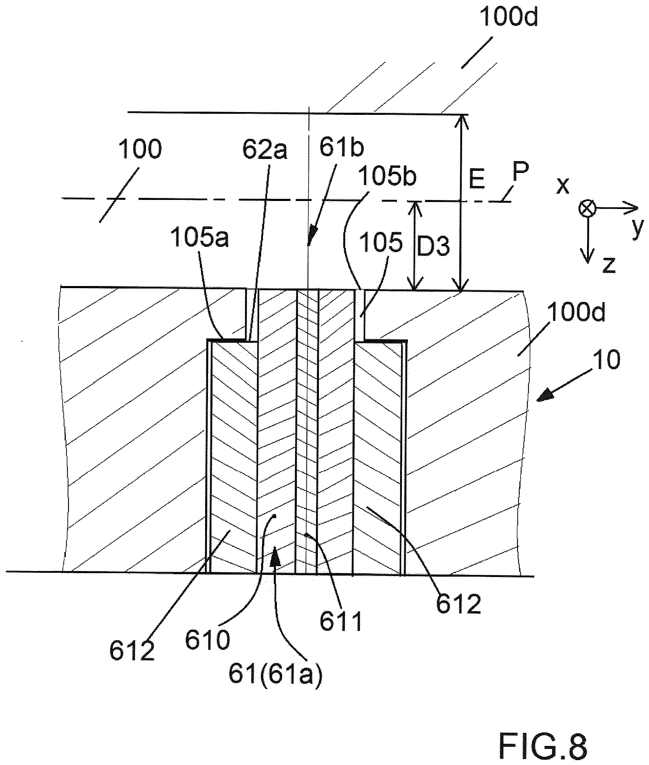

- la figure 8 est une vue en coupe transversale de ce dispositif de tri, dans le plan de coupe VIII-VIII de la figure 7;
- la figure 9 est une vue en coupe transversale d'une deuxième variante d'un dispositif de sélection ;
- la figure 10 est une vue en coupe transversale du canal de transport sur lequel ont été schématisés deux cônes de détection de fluorescence C1 et C2 ;
- la figure 11 représente deux exemples de chronogrammes de deux signaux de détection de fluorescence d'un dispositif de l'invention ;
- la figure 12 représente de manière schématique un exemple de signature de fluorescence (PIC) dans un signal de détection de fluorescence ;
- la figure 13 est un exemple d'histogramme du maximum de l'intensité de fluorescence détectée en fond de canal de transport dans un dispositif du type de celui de la figure 1 ;
- la figure 14 est un exemple d'histogramme des durées (D) des signatures de fluorescence détectée latéralement dans un dispositif du type de celui de la figure 1 ;
- la figure 15 est un exemple d'histogramme des durées (D) des signatures de fluorescence détectée en fond de canal de transport dans un dispositif du type de celui de la figure 1 ;
- la figure 16 représente trois exemples de chronogrammes respectivement des deux signaux de détection de fluorescence et du signal pour la commande des moyens de sélection du dispositif de la figure 1 qui est généré automatiquement à partir de ces deux signaux de détection de fluorescence.

### Description détaillée

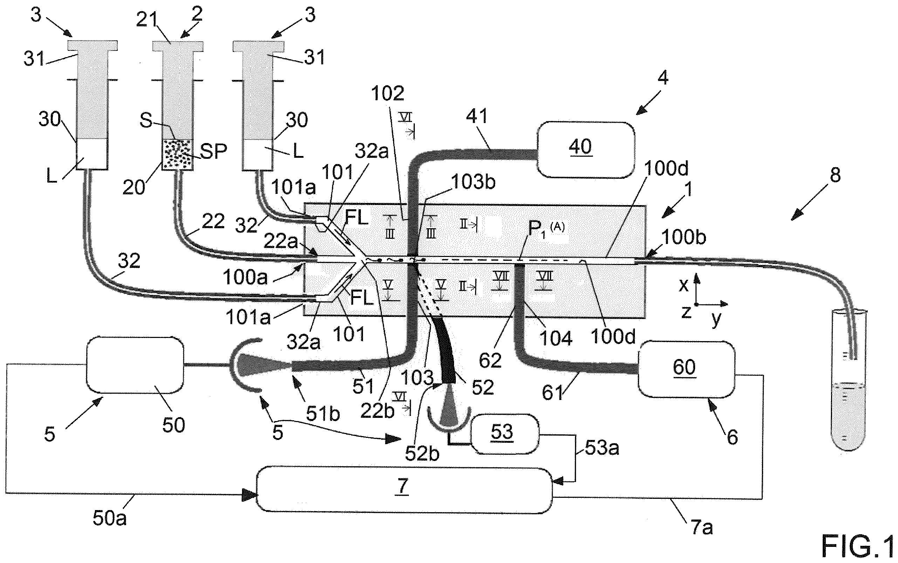

On a représenté sur la figure 1, une première variante de réalisation d'un dispositif permettant de discriminer et de sélectionner des spermatozoïdes en fonction de leur chromosome X ou Y.

Ce dispositif de l'invention peut être utilisé pour sélectionner in vitro des spermatozoïdes X ou Y, et plus particulièrement peut être utilisé dans le domaine de la reproduction animale pour sélectionner *in vitro* tout type de spermatozoïdes X ou Y, et de manière non limitative et non exhaustive, des spermatozoïdes de bovins, de porcins, d'ovins, d'équidés, de caprins, de lapins.

Ce dispositif comporte :
- une puce microfluidique 1 comportant un canal de transport microfluidique 100 principal,
- des moyens d'injection 2 permettant d'injecter, dans le canal de transport microfluidique 100 principal, une solution S contenant un échantillon de spermatozoïdes SP à discriminer et à sélectionner,
- des moyens (3, 101) de focalisation hydrodynamique permettant d'injecter, dans le canal de transport microfluidique 100 principal, un liquide L (fluide de compression) de manière à obtenir une focalisation hydrodynamique des spermatozoïdes dans le canal de transport microfluidique 100 principal,
- des moyens 4 d'excitation de la fluorescence des spermatozoïdes SP circulant dans le canal microfluidique 100 principal,
- des moyens 5 de détection de la fluorescence émise par les spermatozoïdes circulant dans le canal de transport microfluidique 100 principal,
- des moyens de sélection 6, comportant une source de rayonnement électromagnétique 60, et permettant d'altérer de manière sélective les spermatozoïdes circulant dans le canal de transport microfluidique principal 100, au moyen du rayonnement électromagnétique émis par la source 60,
- des moyens électroniques de traitement 7, qui permettent de discriminer automatiquement les spermatozoïdes en fonction de de leur chromosome X ou Y, à partir de la détection de fluorescence réalisée par les moyens de détection 5, et qui dans cette variante particulière de réalisation permettent également de commander automatiquement ladite source de rayonnement électromagnétique 60,
- des moyens de collecte 8 permettant de collecter tous les spermatozoïdes en sortie du canal de transport microfluidique principal 100.

### Puce microfluidique 1- Canal de transport microfluidique principal 100

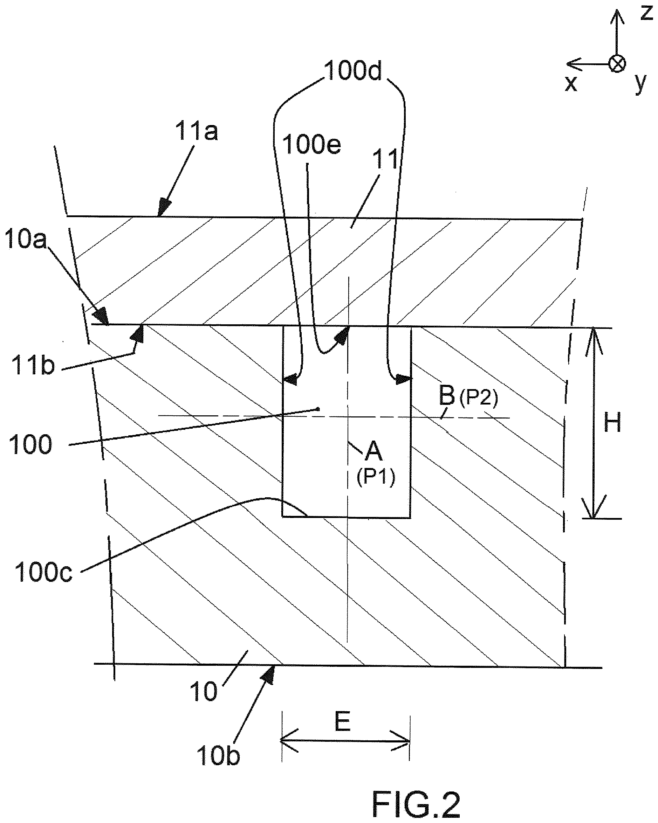

En référence à la figure 2, la puce microfluidique 1 comprend un substrat rigide 10, ayant une face avant 10a et une face arrière 10b, et une plaque 11 fixée contre la face avant 10a du substrat 10.

En référence à la figure 1, dans cette variante particulière de réalisation, le canal de transport microfluidique principal 100 est rectiligne et s'étend suivant la direction longitudinale Y correspondant à la direction de déplacement des spermatozoïdes dans le canal microfluidique 100. Ce canal microfluidique 100 traverse de part en part le substrat 10 et comporte une ouverture d'admission 100a et une ouverture d'évacuation 100b.

Dans une autre variante, tout ou partie de ce canal microfluidique principal 100 pourrait ne pas être rectiligne.

Plus particulièrement, en référence à la figure 2, la section transversale (dans un plan (X,Z)) du canal de transport microfluidique principal 100 est rectangulaire, de hauteur H mesurée dans la direction Z perpendiculaire à la face supérieure 10a plane du substrat 10, et de largeur E mesurée dans la direction transversale X, parallèle à la face supérieure 10a plane du substrat 10 et perpendiculaire à la direction longitudinale Y du canal microfluidique. La hauteur H du canal 100 est supérieure à la largeur E du canal microfluidique100.

De préférence, la largeur E du canal est inférieure à 1mm, et plus préférentielle encore inférieure à 100µm. La hauteur H du canal peut également être inférieure à 1mm.

La structure et la technique de fabrication de la puce microfluidique 1 comportant ledit canal de transport microfluidique 100 sont sans importance et ne sont pas limitatives de l'invention.

A titre d'exemple uniquement, dans la variante de réalisation de la figure 2, le canal microfluidique principal 100 est délimité par une rainure ayant une forme en U en section transversale et réalisée dans la face supérieure 10a du substrat 10, et par la face arrière 11b de la plaque 11. La paroi de fond 100c de la rainure forme la paroi de fond du canal microfluidique 100 ; les deux parois 100d de la rainure qui sont parallèles, et qui sont transversales, et plus particulièrement perpendiculaires à la paroi de fond 100c, forment les deux parois longitudinales 100d du canal microfluidique 100 ; la partie de la face inférieure 11b de la plaque 11, située au droit de la rainure en U, forme la paroi supérieure 100e du canal microfluidique 100.

Cette section transversale rectangulaire du canal microfluidique 100 permet de contribuer à l'orientation spatiale des spermatozoïdes dans le canal 100, notamment en combinaison avec la focalisation hydrodynamique. En particulier, les spermatozoïdes ayant une forme non sphérique et aplatie, la mise en œuvre d'un canal microfluidique 100 à section transversale rectangulaire contribue à une orientation spatiale des spermatozoïdes avec leurs faces aplaties F de plus grande surface orientées sensiblement parallèlement au plan (Y,Z), c'est-à-dire sensiblement parallèles au plan des deux parois longitudinales 100d du canal microfluidique 100. Il revient à l'homme de l'art de sélectionner judicieusement et de manière connue en soi les dimensions du canal microfluidique 100, et notamment le ratio H/E.

Il convient toutefois de souligner que l'invention n'est pas limitée à la mise en œuvre d'un canal de transport 100 ayant une section transversale rectangulaire, ledit canal de transport 100 pouvant plus généralement avoir une géométrie différente en section transversale contribuant à l'orientation spatiale susvisée des spermatozoïdes.

Plus généralement, on pourra ainsi mettre en œuvre un canal de transport 100 comportant un plan de symétrie principal P1 parallèle à la direction de transport Y, et comportant, dans un plan X,Z perpendiculaire à la direction de transport, une section transversale, qui est symétrique, qui est caractérisée par un axe de symétrie principal A (figure 2), et qui présente une dimension transversale maximale H, mesurée le long de cet axe de symétrie principal A, et une dimension transversale minimale E, inférieure à la dimension transversale maximale H, et mesurée le long d'un axe transverse secondaire B (figure 2) perpendiculaire à l'axe de symétrie principal A. Par exemple et de manière non limitative et non exhaustive, le canal de transport 100 peut avoir en section transversale une forme ovale, elliptique, ...

Le substrat 10 est réalisé dans un matériau, qui est chimiquement inerte. Par exemple, mais non nécessairement, le matériau du substrat 10 est choisi de manière à pouvoir subir une gravure physique ou chimique, par exemple une gravure par plasma. Plus particulièrement, mais de manière non limitative de l'invention, le substrat est par exemple en silicium ou en arséniure de gallium. Dans ce cas, la rainure (100c,100d) de hauteur H et de largeur E est avantageusement réalisée par une gravure anisotrope de la face supérieure 10a du substrat 10. Le substrat avec la rainure (100c,100d) peut également être produit par tout autre procédé de fabrication connu et par exemple par impression 3D ou par une technologie dite «hot embossing». Le substrat peut être également être réalisé dans un matériau polymère.

La plaque 11 est dans un matériau qui est chimiquement inerte, et peut être opaque ou transparente. La plaque 11 est par exemple en verre ou en plastique. Elle est fixée au substrat 10 par tout moyen, et par exemple par collage anodique ou thermocompression.

### Moyens d'injection 2 des spermatozoïdes dans le canal microfluidique

Les moyens d'injection 2 ont pour fonction d'injecter, dans le canal microfluidique 100 principal, une solution S contenant un échantillon de spermatozoïdes à sélectionner.

Plus particulièrement, dans la variante de la figure 1, ces moyens d'injection 2 comportent une seringue 20, qui est remplie avec une solution S contenant l'échantillon de spermatozoïdes SP à sélectionner, et qui est associée à un système d'injection automatique 21, qui peut par exemple être de type pousse-seringue ou de type pompe péristaltique. La sortie de la seringue 20 est couplée à un tube capillaire 22, dont la partie distale 22a a été insérée dans le canal microfluidique 100 par l'ouverture d'admission 100a de ce canal 100. Le tube capillaire 22 est un tube souple ou rigide, dont la section transversale est de préférence adaptée à la section du canal microfluidique 100. Ces moyens d'injection 2 particuliers susvisés peuvent être remplacés par tout système d'injection équivalent permettant d'injecter, dans le canal microfluidique 100 principal, avec un débit contrôlé, une solution S contenant un échantillon de spermatozoïdes à sélectionner.

Plus particulièrement, le tube capillaire 22 est de préférence fixé au substrat 10, par tout moyen, et notamment par collage.

Les spermatozoïdes SP sont dilués dans la solution tampon S, qui est compatible biologiquement avec les spermatozoïdes, et par exemple dans une solution aqueuse comportant 30g/L de TRIS (trishydroxyméthylaminométhane), 17,25 g/L d'acide citrique monohydraté et 12,5 g/L de fructose dans de l'eau à un pH d'environ 7. De nombreuses autres solutions tampon S connues de l'homme du métier peuvent être utilisées. On pourra sur ce point se référer par exemple à l'enseignement de la demande de brevet internationale WO2004/088283.

Plus particulièrement, l'ADN des spermatozoïdes SP contenus dans la solution tampon S a été marqué, de manière connue en soi, au moyen d'au moins un fluorochrome, qui peut fluorescer lorsqu'il est associé à l'ADN. Parmi les fluorochromes couramment utilisés pour marquer les spermatozoïdes, on peut citer à titre d'exemples non limitatifs et non exhaustifs : les fluorochromes de type bisbenzimide, et notamment les fluorochromes Hoechst (Hoechst 33342, Hoechst 33258, ...), le bromure d'éthidium, les fluorochromes SYBR tel que le SYBR-14 .

De nombreux autres fluorochromes connus de l'homme du métier peuvent être utilisés. En particulier, pour de plus amples détails sur la réalisation d'une solution tampon S comportant des spermatozoïdes marqués au moyen de fluorochromes, on pourra se référer par exemple à l'enseignement de la demande de brevet internationale WO2004/088283.

En fonctionnement, le système d'injection 21 pousse la solution tampon S contenant les spermatozoïdes SP, de manière à la faire sortir par l'ouverture distale 22b du capillaire 22, et à l'injecter dans le canal microfluidique 100 avec un débit contrôlé automatiquement, et de préférence constant. On a pu vérifier que cette injection de la solution tampon S contenant les spermatozoïdes SP dans le canal microfluidique 100 n'affectait pas la fertilité, et notamment la motilité des spermatozoïdes.

### Moyens (3, 101) de focalisation hydrodynamique des spermatozoïdes

Afin de permettre la mise en œuvre d'une focalisation hydrodynamique des spermatozoïdes SP dans le canal microfluidique 100, la puce microfluidique 1 comporte deux canaux microfluidiques secondaires 101, qui de manière usuelle sont formés de part et d'autre du canal de transport microfluidique 100 (Figure 1). Chaque canal microfluidique secondaire 101 comporte une ouverture d'admission 101a et débouche, à l'opposé de l'ouverture d'admission 101a, latéralement dans le canal microfluidique principal 100.

La sortie du tube capillaire 22 est positionnée en amont de la zone de jonction entre les canaux microfluidiques secondaires 101 et le canal de transport microfluidique 100, la partie distale 22a du tube capillaire 22 pouvant être insérée plus ou moins profondément dans le canal de transport 100.

Plus particulièrement, et de manière similaire à ce qui a été décrit précédemment pour le canal microfluidique principal 100, chaque canal microfluidique secondaire 101 est délimité d'une part par une rainure en U gravée dans la face supérieure du substrat 10, et d'autre part par la face inférieure 11b de la plaque 11.

Les moyens de focalisation hydrodynamique comportent pour chaque canal secondaire 101, des moyens d'injection 3 sous la forme d'une seringue 30, qui est remplie avec une solution L, et qui est associée à un système d'injection 31 automatique, par exemple de type pousse-seringue ou de type pompe péristaltique ou de tout autre moyen d'actionnement de liquide. La sortie de la seringue 30 est couplée à un tube capillaire 32, dont la partie distale 32a a été insérée dans le canal microfluidique 100 par l'ouverture d'admission 101a d'un canal secondaire 101. Chaque tube capillaire 32 est un tube souple, dont la section transversale est de préférence adaptée à la section du canal secondaire 101. Plus particulièrement, chaque tube capillaire 32 est de préférence fixé au substrat 10, par tout moyen, et notamment par collage.

Le liquide utilisé pour les solutions L est de préférence, mais non nécessairement, identique à celui utilisé pour la solution tampon S contenant les spermatozoïdes SP.

En fonctionnement, chaque système d'injection 31 pousse chaque solution L de manière à l'injecter dans le canal microfluidique secondaire 101 correspondant avec un débit contrôlé automatiquement, et de préférence constant.

De manière connue en soi, les débits de chaque solution L et de la solution tampon S contenant les spermatozoïdes SP sont contrôlés automatiquement, de manière à créer dans le canal microfluidique 100a, deux flux laminaires FL à vitesse élevée formés par chaque solution L, de part et d'autre du flux central plus lent formé par la solution S contenant les spermatozoïdes SP. Ces flux laminaires FL permettent, de manière connue, d'entraîner les spermatozoïdes SP dans le canal microfluidique principal 100 en leur faisant subir une focalisation hydrodynamique, de type 2D, qui aboutit sensiblement à aligner les spermatozoïdes SP les uns derrière les autres, avec un alignement des spermatozoïdes SP sensiblement dans un plan de focalisation hydrodynamique longitudinal parallèle au plan de symétrie principal P1 du canal de transport 100. En outre la section rectangulaire ou équivalente du canal de transport 100 permet d'obtenir une orientation spatiale des spermatozoïdes avec leur face aplatie de plus grande surface orientée sensiblement parallèlement au plan de symétrie principal P1 du canal de transport 100, c'est-à-dire sensiblement parallèlement au plan des deux parois longitudinales 100d du canal microfluidique 100 dans la variante des figures 1 et 2.

La position de ce plan de focalisation hydrodynamique des spermatozoïdes entre les deux parois longitudinales 100d du canal 100 dépend notamment de la différence de débit entre les deux flux laminaires FL de liquide L. Lorsque ces débits sont égaux, le plan de focalisation hydrodynamique des spermatozoïdes est sensiblement centré entre les deux parois longitudinales 100d du canal 100 (Figure 2), c'est-à-dire correspond sensiblement au plan de symétrie principal P1 du canal de transport 100. Dans le cadre de l'invention, le plan de focalisation hydrodynamique des spermatozoïdes peut néanmoins être décentré entre les deux parois longitudinales 100d du canal 100 par rapport au plan de symétrie principal P1.

L'invention n'est pas limitée à une focalisation hydrodynamique 2D des spermatozoïdes SP dans le canal microfluidique principal 100. Il est également possible dans le cadre de l'invention de mettre en œuvre une focalisation hydrodynamique 3D, tel que décrit par exemple dans la demande de brevet internationale WO2011/005776, de manière à aligner les spermatozoïdes sensiblement le long d'un axe de focalisation hydrodynamique longitudinal parallèle à l'axe Y du canal microfluidique 100.

Les moyens d'injection 3 particuliers susvisés peuvent être remplacés par tout système d'injection équivalent permettant d'injecter, dans le canal microfluidique 100 principal, avec un débit contrôlé, les solutions L pour obtenir de manière connue en soi ladite focalisation hydrodynamique 2D ou 3D des spermatozoïdes dans le canal de transport 100.

### Moyens 4 d'excitation de la fluorescence

Les moyens 4 d'excitation de la fluorescence comportent une source 40 de rayonnement électromagnétique, de type source laser, dont la longueur d'onde est adaptée au marqueur (fluorochrome) des spermatozoïdes. Par exemple, et de manière non limitative de l'invention, on met en œuvre un rayonnement électromagnétique d'excitation de fluorescence de longueur d'onde comprise entre 300nm et 400nm, et par exemple plus particulièrement aux alentours de 375 nm lorsque les spermatozoïdes SP ont été marqués avec du Hoechst.

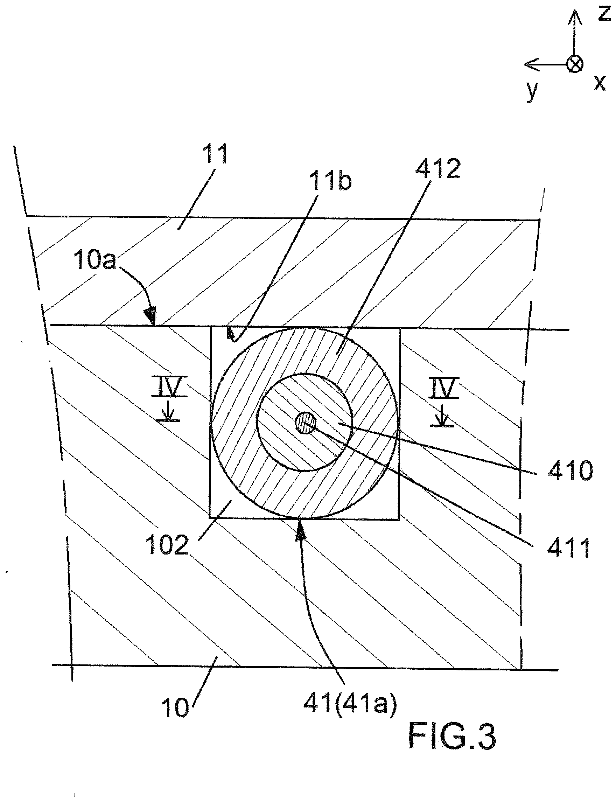

La puce microfluidique 1 comporte un premier passage traversant 102, qui débouche dans le canal microfluidique principal 100 (figures 1 et 3) dans une partie du canal microfluidique principal 100 située en aval de la zone de focalisation hydrodynamique (zone de jonction entre les canaux 100 et 101).

Dans la variante particulière de la figure 1, ce passage traversant 102 est réalisé à travers l'une des parois longitudinales 100d du canal microfluidique 100.

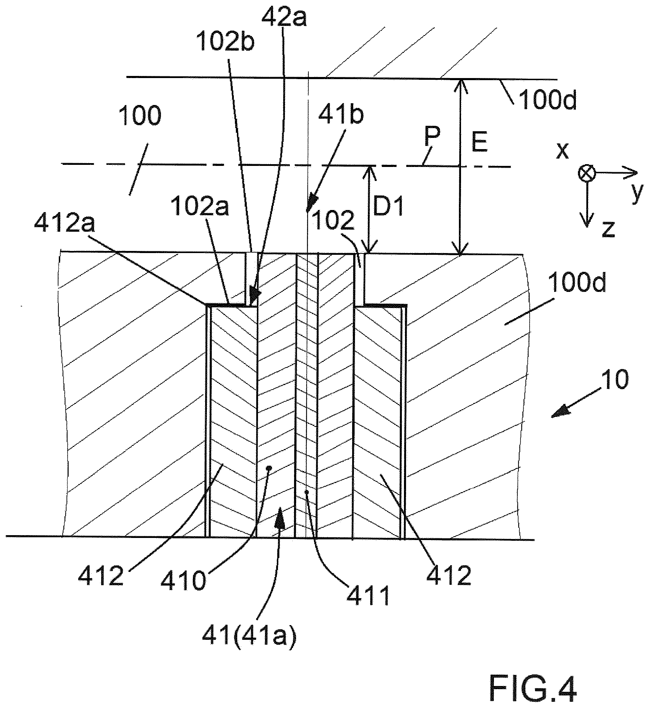

Dans la variante illustrée sur les figures 1 et 4, la distance entre la sortie 102b dans le canal de transport 100 du passage traversant 102 et la paroi opposée 100d du canal de transport 100 correspond à la largeur E du canal de transport 100, et est de préférence inférieure à 1mm, plus préférentiellement inférieure à 100µm.

Ce passage traversant 102 est délimité par une rainure en U gravée dans la face supérieure 10a du substrat 10 et par la face inférieure 11b de la plaque 11. Dans une autre variante, le passage traversant 101 pourrait être percé à travers le substrat 10.

La sortie de la source de rayonnement électromagnétique 40 est couplée à une fibre optique 41 dont la partie distale 41a est insérée dans ce premier passage traversant 102, de telle sorte que l'extrémité d'émission 41b (figure 4) de la fibre optique 41 ne soit pas en saillie dans le canal microfluidique 100, afin de ne pas perturber les écoulements de fluides dans le canal microfluidique 100, et de ce fait de ne pas perturber le positionnement et l'orientation spatiale des spermatozoïdes SP circulant dans le canal microfluidique 100. De préférence, l'extrémité d'émission 41b de la fibre optique 41 est positionnée au plus près de ce canal microfluidique 100, et de préférence affleure le canal microfluidique 100.

Sur cette figure 4, la gaine mécanique de la fibre optique est référencée 412, la gaine optique de la fibre optique est référencée 410, et le cœur de la fibre optique est référencée 411. Dans cette variante, l'extrémité distale de la gaine optique 410 et du cœur 411 de la fibre optique, par laquelle est émis le rayonnement électromagnétique d'excitation, est dénudée. Dans une autre variante, l'extrémité distale de la gaine optique 410 et du cœur 411 de la fibre optique pourrait ne pas être dénudée et être entourée par la gaine mécanique 412 de la fibre optique 41.

Plus particulièrement, dans la variante de la figure 4, le passage traversant 102 est profilé de manière à comporter un épaulement 102a formant une butée de positionnement 102a pour l'extrémité distale 412a de la gaine mécanique 412 de la fibre optique. Il suffit ainsi d'insérer la fibre optique jusqu'à ce que l'extrémité distale 412a de la gaine mécanique 412 soit bloquée par la butée de positionnement 102a, ce qui permet avantageusement un positionnement simple et précis de l'extrémité distale d'émission de la fibre optique 41 par rapport au canal microfluidique 100.

En fonctionnement, lorsque l'extrémité d'émission de la fibre optique 41 affleure le canal de transport 100, le rayonnement électromagnétique d'excitation est émis par la fibre optique 41 directement dans le canal de transport 100. Lorsque l'extrémité d'émission de la fibre optique 41 est positionnée légèrement en retrait dans le passage traversant 102, le rayonnement électromagnétique d'excitation est émis dans ce passage traversant 102 puis pénètre dans le canal de transport 100.

Cette insertion de la fibre optique 41 dans la puce microfluidique 1, à proximité du canal microfluidique 100, permet avantageusement d'amener le rayonnement électromagnétique d'excitation au plus près des spermatozoïdes SP circulant dans le canal microfluidique, ce qui contribue à améliorer les performances de l'excitation de la fluorescence.

Afin également d'améliorer les performances de l'excitation de la fluorescence, la distance D₁ (figure 4), entre la sortie 102b dans le canal de transport 100 du premier passage traversant 102 et le plan de focalisation hydrodynamique (focalisation hydrodynamique 2D) ou l'axe de focalisation hydrodynamique (focalisation hydrodynamique 3D) des spermatozoïdes SP dans le canal de transport 100, est de préférence très faible. On réduit ainsi avantageusement la longueur du trajet du rayonnement électromagnétique d'excitation jusqu'aux spermatozoïdes SP, à travers le liquide transportant les spermatozoïdes dans le canal de transport 100. De préférence, mais non nécessairement cette distance D₁ est inférieure à 1mm, plus préférentiellement inférieure à 100µm, et plus préférentiellement encore inférieure à 50µm.

L'insertion de la fibre optique 41 dans la puce microfluidique 1 permet également d'éviter les risques de désalignement du rayonnement électromagnétique d'excitation par rapport au canal microfluidique 100 lorsque l'on manipule la puce microfluidique 1.

L'invention n'est toutefois pas limitée à la mise en œuvre d'une fibre optique 41 intégrée à la puce et débouchant dans le canal de transport 100. Dans une autre variante, les moyens 4 d'excitation de la fluorescence peuvent comporter une source d'excitation lumineuse (non nécessairement fibrée) qui est extérieure à la puce microfluidique 1 et qui permet un éclairage des spermatozoïdes au moyen d'un rayonnement d'excitation émis à l'extérieur de la puce microfluidique et traversant une paroi du canal de transport.

### Moyens 5 de détection de la fluorescence

Les moyens 5 de détection de la fluorescence comportent une première fibre optique 51 et un photo-détecteur 50, de type photomultiplicateur (PM) qui est adapté pour détecter la longueur d'onde de fluorescence des spermatozoïdes, à savoir par exemple une longueur d'onde comprise entre 400nm et 500nm, et par exemple aux alentours de 460nm lorsque les spermatozoïdes ont été marqués avec un fluorochrome de type Hoechst.

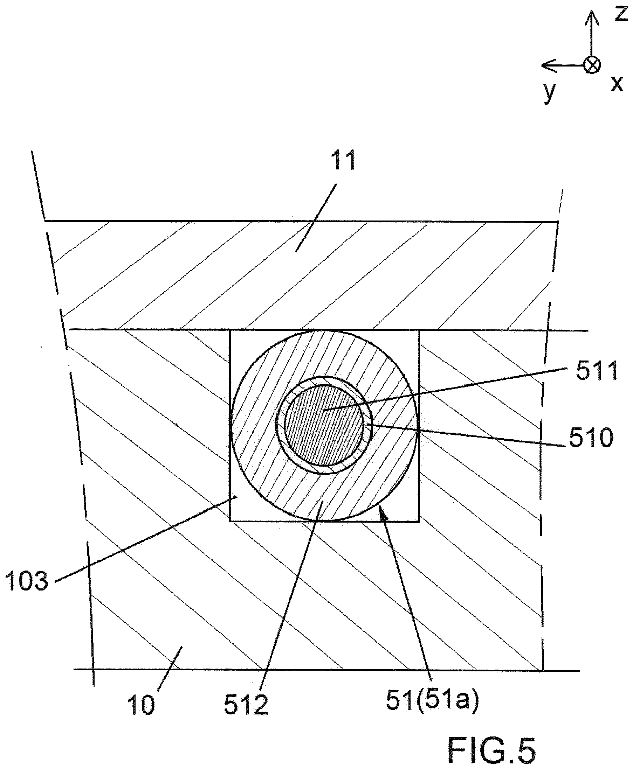

La puce microfluidique 1 comporte un deuxième passage traversant 103, qui débouche dans le canal microfluidique principal 100 (figures 1 et 5) en vis à vis du premier passage traversant 103. Dans la variante particulière de la figure 1, ce passage traversant 103 est réalisé à travers l'autre paroi longitudinale 100d du canal microfluidique 100.

La distance entre la sortie 103b dans le canal de transport 100 du passage traversant 103 et la paroi opposée 100d du canal de transport 100 correspond à la largeur E du canal de transport 100, et est de préférence inférieure à 1mm, plus préférentiellement inférieure à 100µm.

Sur cette figure 5, la gaine mécanique de la fibre optique est référencée 512, la gaine optique de la fibre optique est référencée 510, et le cœur de la fibre optique est référencée 511.

De manière identique à ce qui a été précédemment décrit pour la fibre optique 41, la partie distale 51a de la fibre optique de détection 51 est insérée dans ce deuxième passage traversant 103 de telle sorte que l'extrémité distale de la fibre optique 51 ne soit pas en saillie dans le canal microfluidique 100, et est de préférence positionnée au plus près de ce canal microfluidique 100.

Cette fibre optique 51 permet de détecter la fluorescence émise par les spermatozoïdes dans une direction essentiellement perpendiculaire au plan de symétrie principal P1 du canal de transport 100.

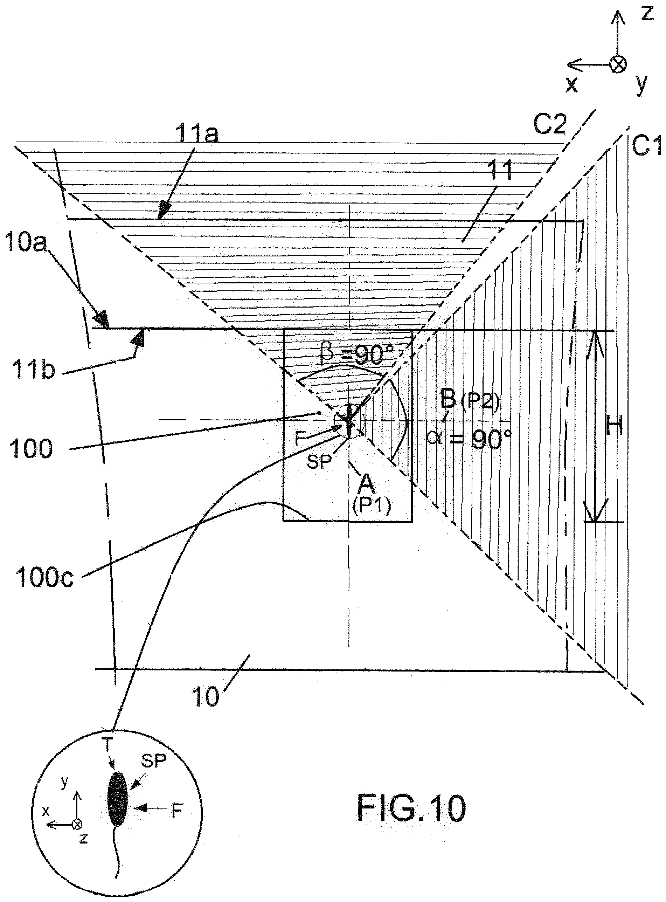

Par les termes «détecter la fluorescence émise par les spermatozoïdes dans une direction essentiellement perpendiculaire au plan de symétrie principal P1 », on signifie que les moyens de détection de la fluorescence, et en l'occurrence dans cette variante particulière de réalisation la première fibre optique 51, permettent de détecter au moins une partie de la fluorescence émise par les spermatozoïdes SP à l'intérieur d'au moins un cône de détection d'au maximum 90°, d'axe perpendiculaire au plan de symétrie principal P1 et dont le sommet est orienté vers le canal de transport 100 (figure10 / cône C1 d'angle α au sommet valant 90°).

Plus particulièrement, mais de manière non limitative de l'invention, ces moyens de détection de la fluorescence, et en l'occurrence dans cette variante particulière de réalisation la première fibre optique 51, permettent de détecter la fluorescence émise par les spermatozoïdes SP au moins dans une direction perpendiculaire au plan de symétrie principal P1.

Il en résulte que lorsque les spermatozoïdes SP sont transportés l'un derrière l'autre dans le canal de transport 100 en étant orientés spatialement de telle sorte que leurs faces principales F de plus grande surface sont sensiblement parallèles au plan de symétrie principal P1 du canal de transport 100, les premiers moyens de détection de la fluorescence, et en l'occurrence dans cette variante particulière de réalisation l'extrémité distale la première fibre optique 51, sont orientés en direction de l'une des deux faces principales F de plus grande surface de chaque spermatozoïde SP passant successivement dans le canal de transport devant lesdits moyens de détection.

L'extrémité d'émission 51b de la fibre de détection 51 est positionnée en vis à vis du photo-détecteur 50, de telle sorte que la lumière (fluorescence) qui est émise dans le canal microfluidique 100, et qui est captée par la fibre 51, est détectée par le détecteur de lumière 50. Le détecteur de lumière 50 délivre un signal électrique 50a caractéristique de l'intensité lumineuse de la fluorescence qui est détectée.

Cette insertion de la fibre optique de détection 51 dans la puce microfluidique 1, à proximité du canal microfluidique 100, permet avantageusement d'améliorer la détection de fluorescence et contribue à une meilleure discrimination entre un spermatozoïde X et un spermatozoïde Y.

Afin également d'améliorer les performances de la détection de fluorescence, la distance entre la sortie 103b dans le canal de transport 100 du deuxième passage traversant 103 (figure 1- ouverture 103b de ce passage 103 débouchant dans le canal de transport 100) et le plan de focalisation hydrodynamique P (focalisation hydrodynamique 2D) ou l'axe de focalisation hydrodynamique (focalisation hydrodynamique 3D) des spermatozoïdes dans le canal de transport 100 est de préférence très faible. On réduit ainsi avantageusement la longueur du trajet du rayonnement de fluorescence, à travers le liquide transportant les spermatozoïdes dans le canal de transport 100. De préférence, mais non nécessairement cette distance est inférieure à 1mm, plus préférentiellement inférieure à 100µm, et plus préférentiellement encore inférieure à 50µm.

Plus particulièrement, afin de collecter un maximum de lumière, la fibre optique de détection 51 peut par exemple être une fibre optique large cœur 511 (figure 5) contrairement à la fibre optique d'excitation 41, dont le cœur 411 (figure 4) est de plus faible diamètre de manière à concentrer spatialement le rayonnement électromagnétique.

En référence à la figure 1, les moyens 5 de détection de la fluorescence comportent également une deuxième fibre optique 52 associée un photo-détecteur 53, de type photomultiplicateur (PM) qui est adapté pour détecter la longueur d'onde de fluorescence des spermatozoïdes, à savoir par exemple une longueur d'onde comprise entre 400nm et 500nm, et par exemple aux alentours de 460nm lorsque les spermatozoïdes ont été marqués avec un fluorochrome de type Hoechst.

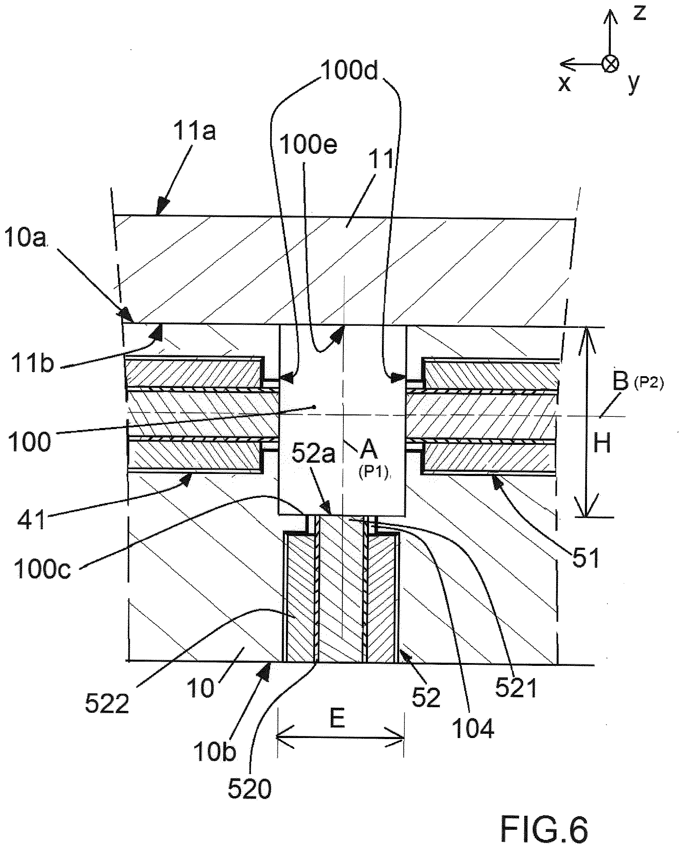

En référence à la figure 6, la puce microfluidique 1 comporte un troisième passage traversant 104, qui est réalisé à travers la paroi de fond 100c du canal microfluidique 100 et qui débouche dans le canal microfluidique principal 100 dans la même zone que les fibres optiques 41 et 51 précitées.

Sur cette figure 6, la gaine mécanique de la fibre optique est référencée 522, la gaine optique de la fibre optique est référencée 520, et le cœur de la fibre optique est référencé 521.

De manière identique à ce qui a été précédemment décrit pour la fibre optique 41, la partie distale 52a de la fibre optique de détection 52 est insérée dans ce troisième passage traversant 104 de telle sorte que l'extrémité distale de la fibre optique 52 ne soit pas en saillie dans le canal microfluidique 100, et est de préférence positionnée au plus près de ce canal microfluidique 100.

L'extrémité d'émission 52b (Figure 1) de la fibre de détection 52 est positionnée en vis à vis du photo-détecteur 53, de telle sorte que la lumière (fluorescence) qui est émise dans le canal microfluidique 100, et qui est captée par la fibre 52 en fond de canal de transport, est détectée par le détecteur de lumière 53. Le détecteur de lumière 53 délivre un signal électrique 53a (Figure 1) caractéristique de l'intensité lumineuse de la fluorescence qui est détectée.

Plus particulièrement, afin de collecter un maximum de lumière, la fibre optique de détection 52 peut par exemple être une fibre optique large cœur 521 (figure 6).

De préférence, cette deuxième fibre optique de détection 52 permet de détecter la fluorescence émise par les spermatozoïdes, dans une direction essentiellement perpendiculaire, et plus particulièrement perpendiculaire, au plan secondaire P2 du canal de transport qui est perpendiculaire au plan de symétrie principal P1.

Par les termes «détecter la fluorescence émise par les spermatozoïdes dans une direction essentiellement perpendiculaire au plan secondaire P2», on signifie que les moyens de détection de la fluorescence, et en l'occurrence dans cette variante particulière de réalisation la première fibre optique 52, permettent de détecter au moins une partie de la fluorescence émise par les spermatozoïdes SP au moins à l'intérieur d'un cône de détection d'au maximum 90°, d'axe perpendiculaire au plan secondaire P2, et dont le sommet est orienté vers le canal de transport 100 (figure10 / cône C2 d'angle β au sommet valant 90°).

Plus particulièrement, mais de manière non limitative de l'invention, ces moyens de détection de la fluorescence, et en l'occurrence dans cette variante particulière de réalisation la deuxième fibre optique 52, permettent de détecter la fluorescence émise par les spermatozoïdes SP au moins dans une direction perpendiculaire au plan secondaire P2.

Il en résulte que lorsque les spermatozoïdes SP sont transportés l'un derrière l'autre dans le canal de transport 100 en étant orientés spatialement de telle sorte que leurs faces principales F de plus grande surface sont sensiblement parallèles au plan de symétrie principal P1 du canal de transport 100, les deuxièmes moyens de détection de la fluorescence, et en l'occurrence dans cette variante particulière de réalisation l'extrémité distale la première fibre optique 52, sont orientés en direction de l'une des deux tranches T de plus faible épaisseur des spermatozoïdes SP passant successivement dans le canal de transport 100 devant lesdits moyens de détection.

L'invention n'est pas limitée à la mise en œuvre de fibres optiques associées à des photo-détecteurs, de type photomultiplicateur (PM), pour détecter la fluorescence émise par les spermatozoïdes, mais les fibres optiques 51 et 52 et les photo-détecteurs peuvent être remplacés par tout autre moyen de détection de fluorescence.

### Moyen de sélection 6

Les moyens de sélection 6 comportent une source 60 de rayonnement électromagnétique, par exemple de type source laser (pulsé ou continu) et une fibre optique 61. La sortie de cette source de rayonnement électromagnétique 61 est couplée à la fibre optique 61.

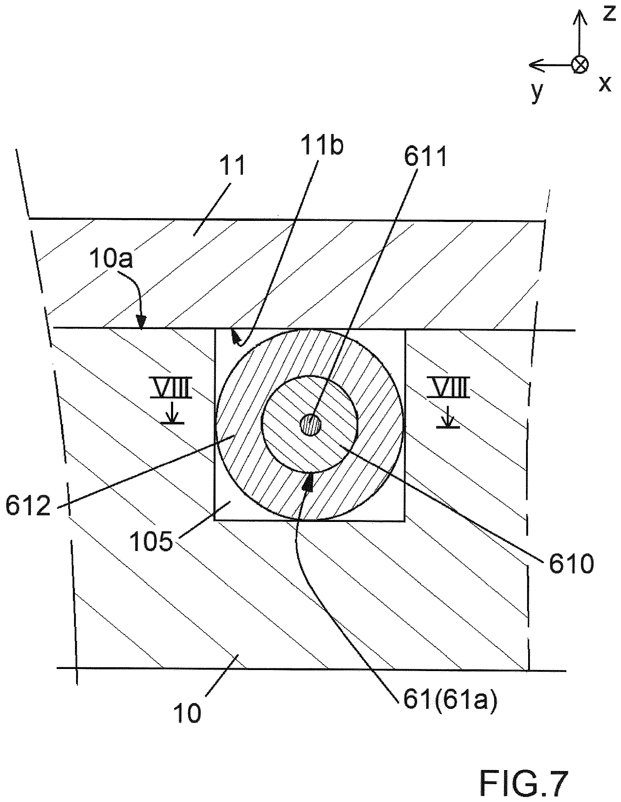

La puce microfluidique 1 comporte un quatrième passage traversant 105, qui débouche dans le canal microfluidique principal 100 (figures 1 et 7) dans une partie du canal microfluidique principal 100 située en aval de la zone de détection de fluorescence.

Dans la variante particulière de la figure 1, ce passage traversant 105 est réalisé à travers l'une des parois longitudinales 100d du canal microfluidique 100.

La distance entre la sortie 105b dans le canal de transport 100 du passage traversant 105 et la paroi opposée 100d du canal de transport 100 correspond à la largeur E du canal de transport 100, et est de préférence inférieure à 1mm, plus préférentiellement inférieure à 100µm.

Sur la figure 7, la gaine mécanique de la fibre optique est référencée 612, la gaine optique de la fibre optique est référencée 610, et le cœur de la fibre optique est référencé 611.

De manière identique à ce qui a été précédemment décrit pour la fibre optique 41, la partie distale 61a de la fibre optique 61 est insérée dans ce quatrième passage traversant 105, de telle sorte que l'extrémité distale d'émission 61b (figure 7) de la fibre optique 61 ne soit pas en saillie dans le canal microfluidique 100, et est de préférence positionnée au plus près de ce canal microfluidique 100.

Lorsqu'elle activée, la source 60 émet dans le canal microfluidique 100 un rayonnement électromagnétique d'altération R, qui a pour fonction d'altérer directement ou indirectement un spermatozoïde SP circulant dans le canal microfluidique principal 100 et passant à travers ce rayonnement électromagnétique d'altération, de manière à ce que ce spermatozoïde ne soit plus fertile. Le spermatozoïde SP traversant le rayonnement électromagnétique d'altération est modifié, de sorte que sa viabilité ou motilité est détériorée de manière suffisante pour que le spermatozoïde ne soit plus fertile, indépendamment du phénomène physique et/ou biologique et/ou chimique occasionnant cette détérioration.

Pour obtenir cette altération, on peut pratiquer une longueur d'onde pour le rayonnement électromagnétique d'altération qui est sélectionnée dans une large gamme spectrale. Plus particulièrement, mais non exclusivement, la longueur d'onde du rayonnement électromagnétique d'altération sera typiquement sélectionnée dans une gamme de longueur d'ondes allant de l'UV à l'Infrarouge.

L'insertion de la fibre optique 61 dans la puce microfluidique 1, à proximité du canal microfluidique 100, permet avantageusement d'amener le rayonnement électromagnétique d'altération des spermatozoïdes au plus près des spermatozoïdes SP circulant dans le canal microfluidique.

En fonctionnement, lorsque l'extrémité d'émission de la fibre optique 61 affleure le canal de transport 100, le rayonnement électromagnétique d'altération est émis par la fibre optique 61 directement dans le canal de transport 100. Lorsque l'extrémité d'émission de la fibre optique 61 est positionnée légèrement en retrait dans le passage traversant 105, le rayonnement électromagnétique d'altération est émis dans ce passage traversant 105 puis pénètre dans le canal de transport 100.

Grâce à l'intégration de la partie distale 61a de la fibre optique 61 dans la puce microfluidique, on évite les risques de désalignement du rayonnement électromagnétique d'altération par rapport au canal microfluidique 100, notamment lorsque l'on manipule la puce microfluidique 1.

L'invention n'est toutefois pas limitée à la mise en œuvre d'une fibre optique 61 intégrée à la puce et débouchant dans le canal de transport 100. Dans une autre variante, les moyens de sélection peuvent par exemple comporter une source de rayonnement qui permet un éclairage pour la sélection des spermatozoïdes au moyen d'un rayonnement émis à l'extérieur de la puce microfluidique et traversant une paroi du canal de transport.

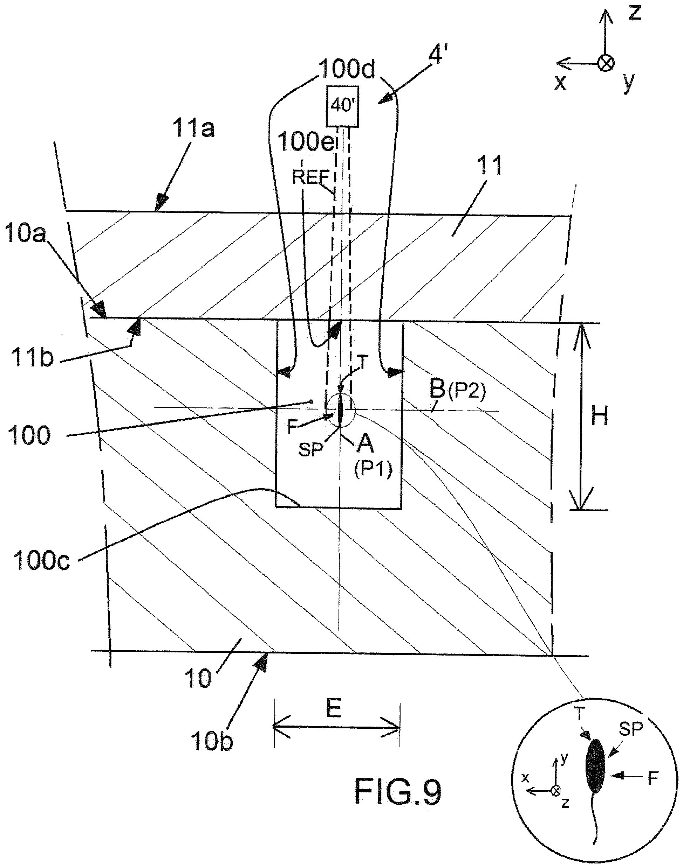

### Autres variante - Figure 9

Dans une autre variante de réalisation schématisée sur la figure 9, les moyens 4 d'excitation de la fluorescence précédemment décrits peuvent être remplacés par des moyens 4' d'excitation de la fluorescence qui comportent une source 40' de rayonnement électromagnétique, par exemple de type source laser, et qui sont adaptés pour émettre un rayonnement électromagnétique d'excitation REF, dont la longueur d'onde est adaptée au marqueur (fluorochrome) des spermatozoïdes. Ce rayonnement électromagnétique d'excitation REF est émis à l'extérieur de la puce microfluidique, et passe à travers la paroi supérieure 11 du canal de transport en direction des spermatozoïdes SP circulant dans ce canal de transport.

Lorsque les spermatozoïdes SP circulent dans le canal de transport 100, de par notamment la géométrie en section transversale du canal de transport, chaque spermatozoïde SP est orienté spatialement de telle sorte que sa face principale F de plus grande surface est sensiblement parallèle au plan de symétrie principal P1 du canal de transport. Dans cette variante de réalisation de la figure 9, on éclaire ainsi principalement l'une des deux tranches T de plus faible épaisseur des spermatozoïdes orientés et circulant dans le canal de transport 100, plutôt que l'une des deux faces F de plus grande surface des spermatozoïdes, à l'inverse de la variante précitée mettant en œuvre un éclairage latéral (moyens 4 d'excitation de la fluorescence susvisés).

En variante, les moyens 4' d'excitation de la fluorescence peuvent également être adaptés pour émettre un rayonnement électromagnétique d'excitation R à l'extérieur de la puce microfluidique, et à travers la paroi de fond 10 du canal de transport. En variante, cet éclairage pourrait également être effectué directement dans le canal de transport au moyen d'une fibre optique, qui est intégrée à la puce microfluidique en étant passée à travers la paroi supérieure 11 ou la paroi de fond 10 du canal de transport.

Plus généralement, pour éclairer principalement la tranche T de plus faible épaisseur des spermatozoïdes orientés et circulant dans le canal de transport 100, on mettra en œuvre un rayonnement électromagnétique d'excitation REF se propageant au moins dans une direction sensiblement parallèle au plan de symétrie principal P1 du canal de transport.

### Moyens électroniques de traitement et de commande 7

Les moyens électroniques de traitement et de commande 7 reçoivent en entrée les deux signaux 50a, 53a de détection de fluorescence délivrés respectivement par les photo-détecteurs 50 et 53, et délivrent en sortie un signal de commande 7a permettant de commander automatiquement ladite source de rayonnement électromagnétique 60, à partir de la détection de fluorescence.

Des essais de détection de fluorescence ont été menés avec de la semence de bovins sexée, marquée au Hoechst 33342, et passée dans un dispositif microfluidique de l'invention. Les considérations ci-après et l'invention ne se limitent pas à la semence de bovins, mais peuvent être généralisées à la semence de tout type de mammifères comportant des chromosomes X ou Y, et notamment à des spermatozoïdes de porcins, d'ovins, d'équidés, de caprins, de lapins.

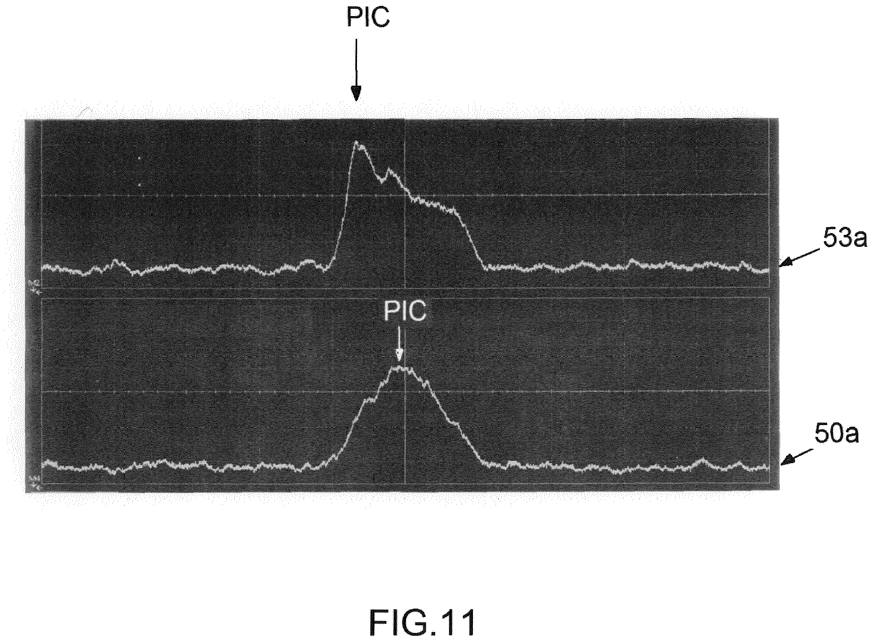

On a représenté sur la figure 11, deux exemples de signaux temporels de détection de fluorescence 50a (détection latérale) et 53a (détection fond de canal) lorsqu'un spermatozoïde SP de mammifère, en l'occurrence d'un bovin, passe dans le canal de transport devant les extrémités distales des deux fibres optiques 51 et 52.

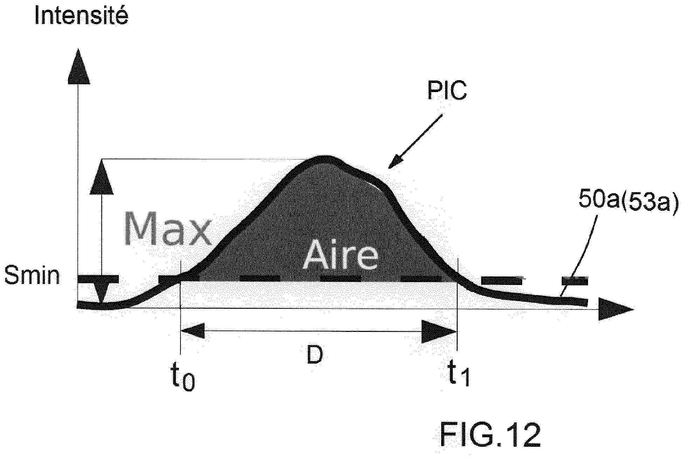

On a également représenté sur la figure 12, un exemple schématique d'un signal temporel de détection de fluorescence 50a ou 53a lorsqu'un spermatozoïde SP de mammifère, en l'occurrence d'un bovin, passe dans le canal de transport devant l'extrémité distale de la fibre optique 51 ou 52. Le passage d'un spermatozoïde devant l'extrémité distale de la fibre optique 51 ou 52 se traduit par une variation temporelle de l'intensité du signal de détection de fluorescence 50a ou 53a. Cette variation temporelle de l'intensité du signal détection de fluorescence 50a ou 53a forme, dans le signal de détection de fluorescence 50a ou 53a, une signature qui est référencée PIC sur les figures, et pendant laquelle l'intensité du signal de détection est constamment supérieure à un seuil minium de fluorescence Sₘᵢₙ prédéfini, et de préférence paramétrable.

Sur le signal de détection de fluorescence 50a ou 53a de la figure 12, on a indiqué la durée D de cette signature PIC au-dessus dudit seuil minium prédéfini Sₘᵢₙ de fluorescence et caractéristique du passage d'un spermatozoïde devant l'extrémité distale de la fibre optique 51 ou 52. Ce seuil minimum Sₘᵢₙ est fixé par l'homme du métier de manière à filtrer le bruit dans le signal de détection 50a ou 53a et à permettre une détection de la signature de fluorescence PIC par les moyens électronique de traitement 7 et une mesure de la durée D de cette signature PIC pendant laquelle l'intensité de fluorescence est au-dessus de ce seuil Sₘᵢₙ. Sur cette figure 12, l'intensité maximale de la signature de fluorescence PIC est référencée « MAX » et l'aire de la signature de fluorescence PIC au-dessus du seuil minimum Sₘᵢₙ est référencée « Aire »

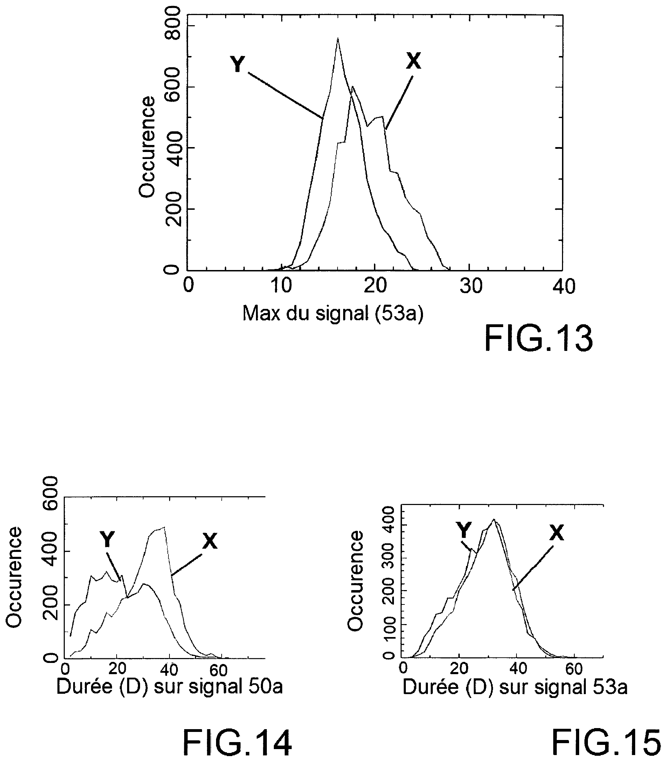

On a représenté sur la figure 13, un exemple d'histogramme du maximum (MAX) de l'intensité de fluorescence sur le détecteur 53 en fond de canal de transport, c'est-à-dire le maximum d'intensité du signal de détection de fluorescence 53a. Cet histogramme fait apparaître deux courbes différentes légèrement décalées en termes d'intensité de fluorescence, qui peuvent être utilisées pour discriminer deux populations différentes de spermatozoïdes X et Y. Il faut toutefois noter qu'une discrimination des spermatozoïdes X et Y basée sur cette seule détection n'est pas optimale, car elle dépend fortement notamment de la qualité de la semence, du protocole de marquage et de fluides de compression utilisés pour orienter les spermatozoïdes dans le canal de transport. En outre le rendement de la détection est faible car seule une fraction des spermatozoïdes peut en pratique être discriminée avec une fiabilité suffisante.

On a représenté sur la figure 14, un exemple d'histogramme des durées D des signatures de fluorescence détectées latéralement (signal 50a) au-dessus dudit seuil prédéfini (Sₘᵢₙ) de fluorescence. Cette figure fait apparaître que les populations X et Y ont des durées d'émission D significativement différentes et que la durée D de la signature de fluorescence, sur le détecteur latéral 50 (signal 50a), est un paramètre qui peut être utilisé pour discriminer les populations X et X de spermatozoïdes, de manière plus fiable et plus discriminante que l'intensité maximale (MAX) de la fluorescence détectée en fond de canal (signal 53a). Des essais réalisés avec différentes espèces de bovins ont montré une reproductibilité du caractère discriminant de ce paramètre de durée D.

On a représenté sur la figure 15, un exemple d'histogramme des durées D des signatures de fluorescence détectées en fond de canal (signal 53a) au-dessus dudit seuil prédéfini (Sₘᵢₙ). Cette figure fait apparaître que les populations X et Y ont des durées d'émission sensiblement identiques et que la durée D de la signature de fluorescence sur le détecteur latéral 53 (signal 53a) n'est pas un paramètre qui peut être utilisé pour discriminer les populations X et X de spermatozoïdes de manière fiable. Cet histogramme permet de confirmer l'importance de l'orientation spatiale des spermatozoïdes dans le canal de transport par rapport aux moyens de détection de fluorescence pour la fiabilité de la durée D de la signature de fluorescence PIC comme moyen de discrimination des spermatozoïdes X/Y.

Dans une variante préférentielle de réalisation, les moyens électroniques de traitement et de commande 7 sont conçus pour traiter en temps réel les deux signaux temporels de détection de fluorescence 50a et 53a et pour délivrer un signal 7a pour la commande des moyens de sélection 6 en fonction de ces deux signaux temporels de détection de fluorescence 50a et 53a.

Un exemple particulier de traitement de ces deux signaux temporels de détection de fluorescence 50a et 53a permettant de détecter automatiquement les spermatozoïdes Y et de commander automatiquement les moyens de sélection 6, pour altérer les autres spermatozoïdes qui n'ont pas été détectés comme spermatozoïdes Y, va à présent être détaillé.

Le traitement du signal temporel de détection de fluorescence 50a (détection latérale) consiste à mesurer, pour chaque signature de fluorescence PIC dans le signal, au moins un paramètre qui est la durée d'émission D de ladite fluorescence détectée par lesdits premiers moyens de détection au-dessus du seuil minium prédéfini (Sₘᵢₙ) de fluorescence susvisé (figure 12).

Plus particulièrement, les moyens électroniques de traitement 7 sont aptes à détecter automatiquement, dans le signal temporel de détection de fluorescence 50a généré par les premiers moyens de détection, l'instant t0 (figure 12) où le signal de détection de fluorescence 50a passe au-dessus du seuil de fluorescence (Smin) prédéfini et l'instant t1 (figure 12) où le signal de détection de fluorescence (50a) repasse au-dessous du seuil de fluorescence (Smin) prédéfini, et sont aptes à mesurer ladite durée (D= t1-t0) de la signature de fluorescence (PIC) comme étant l'intervalle de temps entre ces deux instants détectés.

Les moyens électroniques de traitement et de commande 7 comparent automatiquement ce paramètre (durée D) à un seuil maximum prédéfini et de préférence paramétrable, qui permet une discrimination entre les spermatozoïdes X et Y. Lorsque ce paramètre est en dessous de ce seuil maximum, les moyens électroniques de traitement et de commande 7 détectent automatiquement par exemple qu'il s'agit d'un spermatozoïde Y et permettant ainsi de sélectionner un type de spermatozoïdes.

Le traitement du signal de détection de fluorescence 53a (fond de canal) consiste à mesurer, pour chaque signature de fluorescence PIC, l'intensité maximale de fluorescence MAX et à comparer cette intensité maximale MAX avec un seuil maximum prédéfini Imax qui est choisi de manière à éliminer les signatures de fluorescence non conformes et en particulier, mais pas exclusivement de discriminer notamment les spermatozoïdes collés. En effet en cas de spermatozoïdes collés entre eux, on a pu montrer que l'intensité maximale (MAX) de la fluorescence était anormalement importante.

Lorsque les moyens électroniques de traitement et de commande 7 détectent un spermatozoïde Y (ou respectivement un spermatozoïde X) sur le signal de détection de fluorescence 50a (détection latérale) et détectent en même temps que l'intensité maximale de fluorescence MAX sur le signal de détection 53a (fond de canal) est inférieure au seuil maximum prédéfini Imax, ils déclenchent par exemple un impulsion de commande (signal de commande 7a) permettant d'arrêter temporairement le laser de sélection 60 de manière à ne pas altérer le spermatozoïde Y (ou respectivement un spermatozoïde X) qui a été détecté lorsque celui-ci passe dans le canal de transport devant le laser de sélection.

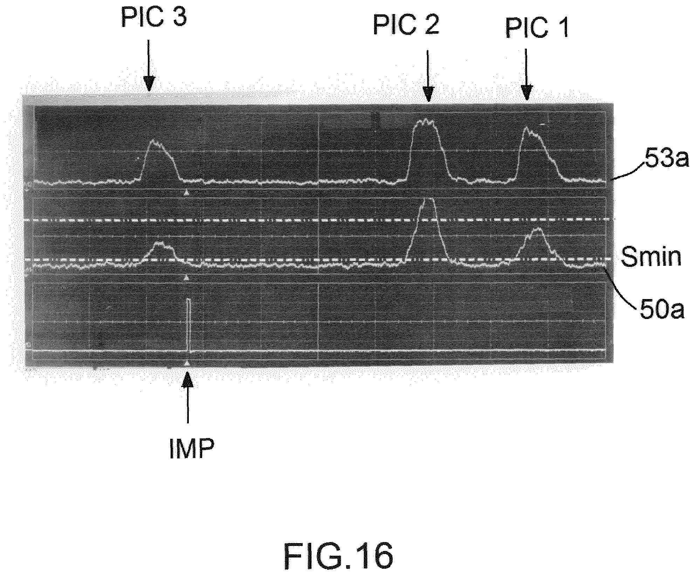

A titre d'exemple, la figure 16 montre un exemple particulier de signal de détection de fluorescence 50a et 53 comportant trois signatures de fluorescence successives référencées respectivement PIC1, PIC2, PIC3.

Seuls les signatures PIC3 dans les deux signaux de détection de fluorescence 50a et 53 permettent une détection d'un spermatozoïde Y et la génération d'une impulsion de commande (IMP) pour l'arrêt temporaire du laser de sélection 60. Pour les signatures précédentes, la signature PIC1 du signal de détection 50a (détection latérale) présente une durée D trop importante pour caractériser un spermatozoïde Y, et la signature PIC2 du signal de détection 53a (détection fond de canal) présente une intensité maximale trop importante (supérieure à Imax).

Dans une autre variante simplifiée de l'invention, les moyens électroniques de traitement et de commande 7 pourraient être conçus pour effectuer une détection uniquement à partir du signal de détection 50a (détection latérale).

Dans les variantes de réalisation qui ont été décrites, la géométrie en section transversale du canal de transport 100 combinée à une focalisation hydrodynamique des spermatozoïdes permet d'orienter spatialement, chaque spermatozoïde SP de telle sorte que sa face principale F de plus grande surface est sensiblement parallèle au plan de symétrie principal P1 du canal de transport 100. L'invention n'est toutefois pas limitée à la géométrie particulière du canal de transport 100 illustré sur les figures annexées, mais s'étend notamment à tout dispositif dans lequel les spermatozoïdes peuvent être transportés dans un canal de transport l'un derrière l'autre en ayant sensiblement la même orientation spatiale. Ainsi, dans une autre variante de réalisation, le canal de transport pourrait par exemple présenter une section transversale circulaire et le dispositif pourrait être équipé, de manière connue soi, d'une buse permettant d'injecter les spermatozoïdes dans ledit canal de transport en leur conférant sensiblement la même orientation spatiale dans le canal de transport.

Dans une autre variante, les moyens de sélection 6 précédemment décrits peuvent être remplacés par des moyens de triage qui permettent de séparer les spermatozoïdes (SP) d'un premier type (par exemple spermatozoïde de type X) des spermatozoïdes (SP) d'un deuxième type (par exemple spermatozoïde de type Y), sans nécessairement altérer l'un des deux types de spermatozoïdes.

## Revendications

1. Dispositif permettant de discriminer des spermatozoïdes (SP) marqués au moyen d'au moins un fluorochrome, lequel dispositif comporte un canal de transport (100), dans lequel peut circuler une solution contenant lesdits spermatozoïdes marqués (SP) dans une direction de transport (Y) prédéfinie, des moyens d'excitation (4 ; 4') permettant d'exciter l'émission par fluorescence des spermatozoïdes marqués (SP) circulant dans ledit canal de transport (100), des moyens de détection (5) de la fluorescence émise par les spermatozoïdes marqués (SP) circulant dans ledit canal de transport (100), et des moyens électroniques de traitement (7) permettant de discriminer les spermatozoïdes (SP) marqués circulant dans le canal de transport à partir de la fluorescence détectée par les moyens de détection de la fluorescence (5), ledit dispositif comporte l'une et/ou l'autre des caractéristiques techniques (a), (b) suivantes :
(a) ledit canal de transport comporte un plan de symétrie principal (P1) parallèle à la direction de transport (Y), et une section transversale, dans un plan (X,Z) perpendiculaire à la direction de transport (Y), qui est symétrique, qui est **caractérisée par** un axe de symétrie principal (A) et présente une dimension transversale maximale (H), mesurée le long de cet axe de symétrie principal (A), et une dimension transversale minimale (E), inférieure à la dimension transversale maximale (H), et mesurée le long d'un axe transverse secondaire (B) perpendiculaire à l'axe de symétrie principal (A), les moyens de détection de fluorescence (5) comportant au moins des premiers moyens de détection (50 ; 51), qui sont aptes à détecter la fluorescence émise par les spermatozoïdes (SP) dans une direction essentiellement perpendiculaire au plan de symétrie principal (P1) du canal de transport, et plus particulièrement au moins dans une direction perpendiculaire au plan de symétrie principal (P1) du canal de transport,
(b) ledit dispositif comportant des moyens permettant le transport des spermatozoïdes (SP) l'un derrière l'autre dans le canal de transport (100) avec sensiblement la même orientation spatiale de chaque spermatozoïde, les moyens de détection de fluorescence (5) comportant au moins des premiers moyens de détection (50 ; 51) qui sont orientés en direction de l'une des deux faces principales (F) de plus grande surface de chaque spermatozoïde (SP) passant successivement dans le canal de transport (100) devant lesdits moyens de détection en étant orienté spatialement,
et le dispositif étant **caractérisé en ce que** les moyens électroniques de traitement (7) sont aptes à discriminer automatiquement les spermatozoïdes (SP) en utilisant au moins la durée (D) de chaque signature de fluorescence (PIC) caractéristique d'un spermatozoïde dans le signal de détection de fluorescence (50a) généré par les premiers moyens de détection (50 ; 51).

2. Dispositif selon la revendication 1, dans lequel les moyens électroniques de traitement (7) sont aptes à mesurer automatiquement la durée (D) de chaque signature de fluorescence (PIC) au-dessus d'un seuil de fluorescence (Smin) prédéfini et à discriminer automatiquement les spermatozoïdes (SP) au moins en utilisant ladite durée (D), et de préférence dans lequel les moyens électroniques de traitement (7) sont aptes à détecter automatiquement dans le signal de détection de fluorescence (50a) généré par les premiers moyens de détection (50 ; 51) l'instant (t0) où le signal de détection de fluorescence (50a) passe au-dessus du seuil de fluorescence (Smin) prédéfini et l'instant (t1) où le signal de détection de fluorescence (50a) repasse au-dessous du seuil de fluorescence (Smin) prédéfini, et sont aptes à mesurer ladite durée (D= t1- t0) de la signature de fluorescence (PIC) comme étant l'intervalle de temps entre ces deux instants détectés.

3. Dispositif selon l'une quelconque des revendications précédentes, dans lequel les moyens de détection de fluorescence comportent en outre des deuxièmes moyens de détection (52 ; 53) aptes à détecter la fluorescence émise par les spermatozoïdes (SP), et dans lequel les moyens électroniques de traitement (7) sont aptes à discriminer automatiquement les spermatozoïdes (SP) circulant dans le canal de transport également à partir de la fluorescence détectée par les deuxièmes moyens de détection (52 ; 53), et plus particulièrement à partir de l'intensité maximale (MAX) de la fluorescence détectée par les deuxièmes moyens de détection (52 ; 53).

4. Dispositif selon la revendication 3, dans lequel les moyens électroniques de traitement (7) sont aptes à discriminer automatiquement les spermatozoïdes (SP) circulant dans le canal de transport au moins à partir de la durée (D) de chaque signature de fluorescence (PIC) caractéristique d'un spermatozoïde dans le signal de détection de fluorescence (50a) généré par les premiers moyens de détection (50 ; 51) et au moins lorsque l'intensité maximale (MAX) de la fluorescence détectée par les deuxièmes moyens de détection (52 ; 53) est inférieure à un seuil maximum (Imax) prédéfini.

5. Dispositif selon la revendication 4, dans lequel les deuxièmes moyens de détection (52 ; 53) sont aptes à détecter la fluorescence émise par les spermatozoïdes (SP) dans une direction essentiellement perpendiculaire au plan secondaire (P2) du canal de transport qui est perpendiculaire au plan de symétrie principal (P1), et plus particulièrement au moins dans une direction perpendiculaire au plan secondaire (P2) du canal de transport

6. Dispositif selon l'une quelconque des revendications précédentes, comportant des moyens (2) permettant d'injecter dans ledit canal de transport (100) une solution (S) contenant des spermatozoïdes (SP) marqués au moyen d'au moins un fluorochrome.

7. Dispositif selon l'une quelconque des revendications précédentes, comportant des moyens de focalisation hydrodynamique (3 ; 101) permettant d'injecter dans le canal de transport (100) un liquide (L) de manière à entraîner les spermatozoïdes (SP) dans le canal de transport (100), en les positionnant sensiblement dans un plan de focalisation hydrodynamique et en les espaçant l'un derrière l'autre, et de préférence dans lequel les moyens de focalisation hydrodynamique (3 ; 101) permettent en combinaison avec le canal de transport (100) d'orienter spatialement avec la même orientation spatiale les spermatozoïdes transportés dans le canal de transport.

8. Dispositif selon l'une quelconque des revendications précédentes, dans lequel les moyens d'excitation (4') sont aptes à éclairer localement les spermatozoïdes marqués (SP) circulant dans ledit canal de transport (100) au moyen d'un rayonnement électromagnétique d'excitation de fluorescence (REF) se propageant au moins dans une direction sensiblement parallèle au plan de symétrie principal (P1) du canal de transport.

9. Dispositif selon l'une quelconque des revendications précédentes, dans lequel les moyens électroniques de traitement permettant de discriminer les spermatozoïdes d'un premier type et les spermatozoïdes d'un deuxième type, et notamment de discriminer les spermatozoïde X et les spermatozoïdes Y, le dispositif comporte des moyens de sélection (6) permettant d'altérer de manière sélective les spermatozoïdes (SP) de l'un des deux types qui circulent dans le canal de transport (100) ou comporte des moyens de triage qui permettent de séparer les spermatozoïdes (SP) d'un premier type des spermatozoïdes (SP) d'un deuxième type.

10. Dispositif selon la revendication 9 dans lequel les moyens de sélection (6) comportent une source de rayonnement électromagnétique (60) qui est apte être commandée automatiquement par les moyens de traitement (7), de manière à altérer de manière sélective les spermatozoïdes (SP) circulant dans le canal de transport (100), au moyen du rayonnement électromagnétique d'altération (R) émis par la source de rayonnement électromagnétique (60).

11. Dispositif selon l'une quelconque des revendications précédentes revendication, dans lequel le canal de transport (100) est un canal microfluidique dont au moins une dimension en section transversale est inférieure à 1mm, et plus particulièrement inférieure à 100µm et/ou dans lequel le canal de transport (100) a une section transversale rectangulaire.

12. Dispositif selon la revendication 11, dans lequel la largeur (E) du canal de transport (100) rectangulaire est inférieure à 1mm, et de préférence inférieure à 100µm.

13. Utilisation du dispositif visé à l'une quelconque des revendications précédentes pour discriminer automatiquement, et le cas échéant pour sélectionner automatiquement ou pour trier automatiquement, *in vitro* des spermatozoïdes.

14. Procédé de discrimination *in vitro* de spermatozoïdes (SP), au cours duquel on injecte, dans le canal de transport (100) du dispositif de l'une quelconque des revendications 1 à 12 une solution (S) contenant les spermatozoïdes (SP) marqués au moyen d'au moins un fluorochrome, on fait circuler dans le canal de transport (100) lesdits spermatozoïdes (SP) les uns derrière les autres en les orientant spatialement avec la même orientation spatiale, et on détecte automatiquement le type de chromosome de chaque spermatozoïde circulant dans le canal de transport (100) en utilisant au moins la durée (D) de la signature de fluorescence (PIC) caractéristique d'un spermatozoïde dans le signal de détection de fluorescence (50a) délivré par les premiers moyens de détection (50 ; 51) du dispositif.

15. Procédé de production d'une semence sexée par sélection ou tri *in vitro* de spermatozoïdes (SP) au moyen du dispositif de l'une quelconque des revendications 1 à 12, au cours duquel on injecte, dans le canal de transport (100) du dispositif une solution (S) contenant les spermatozoïdes (SP) marqués au moyen d'au moins un fluorochrome, on fait circuler dans le canal de transport (100) lesdits spermatozoïdes (SP) les uns derrière les autres en les orientant spatialement avec la même orientation spatiale, on détecte automatiquement le type de chromosome de chaque spermatozoïde circulant dans le canal de transport (100), et notamment si le spermatozoïde est de type X ou Y, en utilisant au moins la durée (D) de la signature de fluorescence (PIC) caractéristique d'un spermatozoïde dans le signal de détection de fluorescence (50a) délivré par les premiers moyens de détection (50 ; 51) du dispositif, et on sélectionne les spermatozoïdes (SP) d'un type donné, notamment en altérant les spermatozoïdes (SP) de l'autre type, ou on trie les spermatozoïdes (SP) en fonction de leur type, et notamment de leur type X ou Y, notamment en séparant les spermatozoïdes (SP) d'un type donné des spermatozoïdes (SP) de l'autre type.

## Patentansprüche

1. Vorrichtung zum Unterscheiden von Spermatozoen (SP), die mit mindestens einem Fluorochrom markiert sind, wobei die Vorrichtung aufweist: einen Transportkanal (100), durch welchen eine die markierten Spermatozoen (SP) enthaltende Lösung in einer vorbestimmten Transportrichtung (Y) fließen kann, Anregungsmittel (4 ; 4') zum Anregen der Fluoreszenzemission von in dem Transportkanal (100) fließenden markierten Spermatozoen (SP), Mittel zum Detektieren (5) der von den in dem Transportkanal (100) fließenden markierten Spermatozoen (SP) emittierten Fluoreszenz, und elektronische Verarbeitungsmittel (7) zum Unterscheiden der in dem Transportkanal fließenden markierten Spermatozoen (SP) anhand der von den Mitteln (5) zum Detektieren der Fluoreszenz erfassten Fluoreszenz, wobei die Vorrichtung eines oder beide der folgenden technischen Merkmale (a), (b) aufweist:
(a) der Transportkanal weist eine Hauptsymmetrieebene (P1) parallel zur Transportrichtung (Y) und einen symmetrischen Querschnitt in einer Ebene (X,Z) senkrecht zur Transportrichtung (Y) auf, der durch eine Hauptsymmetrieachse (A) gekennzeichnet ist und eine maximale Querabmessung (H) aufweist, gemessen entlang dieser Hauptsymmetrieachse (A), und eine minimale Querabmessung (E), die kleiner als die maximale Querabmessung (H) ist und entlang einer zweiten Querachse (B) senkrecht zur Hauptsymmetrieachse (A) gemessen wird, wobei die Fluoreszenzdetektionsmittel (5) mindestens erste Detektionsmittel (50 ; 51) aufweisen, die in der Lage sind, die von den Spermatozoen (SP) emittierte Fluoreszenz in einer Richtung im Wesentlichen senkrecht zur Hauptsymmetrieebene (P1) des Transportkanals, und insbesondere mindestens in einer Richtung senkrecht zu der Hauptsymmetrieebene (P1) des Transportkanals, zu erfassen,
(b) die Vorrichtung weist Mittel auf, die den Transport von Spermatozoen (SP) nacheinander in dem Transportkanal (100) mit im Wesentlichen gleicher räumlicher Ausrichtung jedes Spermatozoons ermöglichen, wobei die Fluoreszenzdetektionsmittel (5) mindestens erste Detektionsmittel (50 ; 51) aufweisen, die in Richtung einer der zwei Hauptflächen (F) mit der größten Oberfläche jedes Spermatozoons (SP), das nacheinander den Transportkanal (100) vor den genannten Erfassungsmitteln unter räumlicher Ausrichtung durchläuft, ausgerichtet sind,
und die Vorrichtung **dadurch gekennzeichnet ist, dass** die elektronischen Verarbeitungsmittel (7) ausgelegt sind, die Spermatozoen (SP) automatisch zu unterscheiden, wobei mindestens die Dauer (D) jeder der für ein Spermatozoon charakteristischen Fluoreszenzsignatur (PIC) in dem Fluoreszenzdetektionssignal (50a), das von den ersten Detektionsmitteln (50 ; 51) erzeugt wurde, verwertet wird.

2. Vorrichtung nach Anspruch 1, wobei die elektronischen Verarbeitungsmittel (7) dazu ausgelegt sind, automatisch die Dauer (D) jeder Fluoreszenzsignatur (PIC) oberhalb eines vordefinierten Fluoreszenzschwellenwerts (Smin) zu messen und automatisch Spermatozoen (SP) zumindest unter Verwendung dieser Dauer (D) zu unterscheiden, und wobei die elektronischen Verarbeitungsmittel (7) bevorzugt dazu ausgelegt sind, automatisch in dem von den ersten Detektionsmitteln (50 ; 51) erzeugten Fluoreszenzdetektionssignal (50a) den Zeitpunkt (t0), an dem das Fluoreszenzdetektionssignal (50a) über den vordefinierten Fluoreszenzschwellenwert (Smin) steigt, und den Zeitpunkt (t1), an dem das Fluoreszenzdetektionssignal (50a) wieder unter den vordefinierten Fluoreszenzschwellenwert (Smin) fällt, zu erfassen, und dazu ausgelegt sind, die Dauer (D= t1- t0) der Fluoreszenzsignatur (PIC) als das Zeitintervall zwischen diesen beiden erfassten Zeitpunkten zu messen.

3. Vorrichtung nach einem der vorhergehenden Ansprüche, wobei die Fluoreszenzdetektionsmittel weiterhin zweite Detektionsmittel (52 ; 53) aufweisen, die in der Lage sind, die von den Spermatozoen (SP) emittierte Fluoreszenz zu detektieren, und wobei die elektronischen Verarbeitungsmittel (7) in der Lage sind, die in dem Transportkanal fließenden Spermien (SP) auch anhand der von den zweiten Detektionsmitteln (52; 53) detektierten Fluoreszenz, und insbesondere anhand der maximalen Intensität (MAX) der von den zweiten Detektionsmitteln (52; 53) detektierten Fluoreszenz, automatisch zu unterscheiden.

4. Vorrichtung nach Anspruch 3, wobei die elektronischen Verarbeitungsmittel (7) in der Lage sind, die in dem Transportkanal fließenden Spermatozoen (SP) zumindest anhand der Dauer (D) jeder für ein Spermatozoon charakteristischen Fluoreszenzsignatur (PIC) in dem von den ersten Detektionsmitteln (50 ; 51) erzeugten Fluoreszenzdetektionssignal (50a) automatisch zu unterscheiden und zumindest dann, wenn die maximale Intensität (MAX) der von den zweiten Detektionsmitteln (52; 53) detektierten Fluoreszenz niedriger als ein vordefinierter maximaler Schwellenwert (Imax) ist.

5. Vorrichtung nach Anspruch 4, wobei die zweiten Detektionsmittel (52; 53) in der Lage sind, die von den Spermatozoen (SP) emittierte Fluoreszenz in einer Richtung zu detektieren, die im Wesentlichen senkrecht zur Sekundärebene (P2) des Transportkanals ist, die senkrecht zu der Hauptsymmetrieebene (P1) ist, und insbesondere mindestens in einer Richtung senkrecht zu der Sekundärebene (P2) des Transportkanals.

6. Vorrichtung nach einem der vorhergehenden Ansprüche, welche Mittel (2) zum Einspritzen einer Lösung (S), welche mit mindestens einem Fluorochrom markierte Spermatozoen (SP) enthält, in den genannten Transportkanal (100) aufweist.

7. Vorrichtung nach einem der vorhergehenden Ansprüche, mit hydrodynamischen Fokussiermitteln (3; 101) zum Einspritzen einer Flüssigkeit (L) in den Transportkanal (100), um die Spermatozoen (SP) in dem Transportkanal (100) mitzuführen, wobei diese im Wesentlichen in einer hydrodynamischen Fokussierebene angeordnet werden und hintereinander beabstandet angeordnet werden, und wobei die hydrodynamischen Fokussiemittel (3 ; 101) bevorzugt in Kombination mit dem Transportkanal (100) eine räumliche Orientierung der in dem Transportkanal transportierten Spermatozoen mit der gleichen räumlichen Orientierung ermöglichen.

8. Vorrichtung nach einem der vorhergehenden Ansprüche, bei der die Anregungsmittel (4') in der Lage sind, die in dem Transportkanal (100) fließenden markierten Spermatozoen (SP) mittels elektromagnetischer Fluoreszenzanregungsstrahlung (REF), die sich zumindest in einer zur Hauptsymmetrieebene (PI) des Transportkanals im Wesentlichen parallelen Richtung ausbreitet, lokal zu bestrahlen.

9. Vorrichtung nach einem der vorhergehenden Ansprüche, wobei die elektronischen Verarbeitungsmittel es ermöglichen, zwischen Spermatozoen eines ersten Typs und Spermatozoen eines zweiten Typs zu unterscheiden, und insbesondere zwischen Spermatozoen X und Spermatozoen Y zu unterscheiden, wobei die Vorrichtung Selektionsmittel (6) zum selektiven Verändern der Spermatozoen des einen der beiden durch den Transportkanal (100) fließenden Typen von Spermatozoen (SP) aufweist, oder Sortiermittel zum Trennen von Spermatozoen (SP) eines ersten Typs von Spermatozoen (SP) eines zweiten Typs aufweist.

10. Vorrichtung nach Anspruch 9, wobei die Selektionsmittel (6) eine elektromagnetische Strahlungsquelle (60) aufweisen, die in der Lage ist, von den Verarbeitungsmitteln (7) automatisch derart gesteuert zu werden, dass die in dem Transportkanal (100) fließenden Spermatozoen (SP) mittels einer von der elektromagnetischen Strahlungsquelle (60) emittierten verändernden elektromagnetischen Strahlung (R) selektiv verändert werden.

11. Vorrichtung nach einem der vorhergehenden Ansprüche, wobei der Transportkanal (100) ein Mikrofluidkanal ist, bei dem mindestens eine Querschnittsabmessung kleiner als 1 mm ist und insbesondere kleiner als 100 µm ist, und/oder wobei der Transportkanal(100)einen rechteckigen Querschnitt aufweist.

12. Vorrichtung nach Anspruch 11, wobei die Breite (E) des rechteckigen Transportkanals (100) kleiner als 1 mm ist, bevorzugt kleiner als 100 µm.

13. Verwendung der Vorrichtung nach einem der vorhergehenden Ansprüche zur automatischen Unterscheidung und wahlweise zur automatischen Auswahl oder automatischen Sortierung von Spermatozoen *in vitro.*

14. Verfahren zur *in* vitro-Unterscheidung von Spermatozoen (SP), bei dem in den Transportkanal (100) der Vorrichtung nach einem der Ansprüche 1 bis 12 eine Lösung (S), die mit mindestens einem Fluorochrom markierte Spermatozoen (SP) enthält, eingespritzt wird, die Spermatozoen (SP) dazu gebracht werden, in dem Transportkanal (100) hintereinander unter räumlicher Ausrichtung mit der gleichen räumlichen Ausrichtung zu fließen, und der Chromosomentyp jedes in dem Transportkanal (100) fließenden Spermatozoons automatisch anhand mindestens der Dauer (D) der für ein Spermatozoon charakteristischen Fluoreszenzsignatur (PIC) in dem Fluoreszenzdetektionssignal (50a), das von den ersten Detektionsmitteln (50 ; 51) der Vorrichtung ausgegeben wird, automatisch detektiert wird.

15. Verfahren zur Herstellung eines geschlechtsspezifischen Samens durch *in*vitro-Selektion oder -Sortierung von Spermatozoen (SP) mittels der Vorrichtung nach einem der Ansprüche 1 bis 12, wobei in den Transportkanal (100) der Vorrichtung nach einem der Ansprüche 1 bis 12 eine Lösung (S), die mit mindestens einem Fluorochrom markierte Spermatozoen (SP) enthält, eingespritzt wird, die Spermatozoen (SP) dazu gebracht werden, in dem Transportkanal (100) hintereinander unter räumlicher Ausrichtung mit der gleichen räumlichen Ausrichtung zu fließen, der Chromosomentyp jedes in dem Transportkanal (100) strömenden Spermatozoons, und insbesondere, ob das Spermatozoon vom X-Typ oder Y-Typ ist, automatisch detektiert wird, indem mindestens die Dauer (D) der für ein Spermatozoon charakteristischen Fluoreszenzsignatur (PIC) in dem von den ersten Detektionsmitteln (50 ; 51) der Vorrichtung ausgegebenen Fluoreszenz-Detektionssignal (50a) verwendet wird, und Spermatozoen (SP) eines vorgegebenen Typs selektiert werden, insbesondere unter Veränderung der Spermatozoen (SP) des anderen Typs, oder die Spermatozoen (SP) entsprechend ihrem Typ, insbesondere dem X-Typ oder Y-Typ, sortiert werden, insbesondere unter Trennung der Spermatozoen (SP) eines vorgegebenen Typs von den Spermatozoen (SP) des anderen Typs.

## Claims

1. Device for discriminating spermatozoa (SP) labeled with at least one fluorochrome, which device comprises a transport channel (100), into which a solution containing said labeled spermatozoa (SP) can circulate in a predefined transport direction (Y), excitation means (4; 4') allowing the stimulation of fluorescence emission of the labeled spermatozoa (SP) circulating in said transport channel (100), means (5) for detecting the fluorescence emitted by the labeled spermatozoa (SP) circulating in said transport channel (100), and electronic processing means (7) for discriminating labeled spermatozoa (SP) circulating in the transport channel from the fluorescence detected by the means of fluorescence detection (5), said device comprises one and/or the other of the following technical characteristics (a), (b) :
(a) said transport channel comprises a main plane of symmetry (P1) parallel to the transport direction (Y), and a cross section in a plane (X, Z) perpendicular to the transport direction (Y), which is symmetrical, which is **characterized by** a main axis of symmetry (A) and has a maximum cross-sectional dimension (H), measured along this main axis of symmetry (A), and a minimum cross-sectional dimension (E), smaller than the maximum cross-sectional dimension (H), and measured along a secondary cross-sectional axis (B) perpendicular to the main axis of symmetry (A), the fluorescence detection means (5) comprising at least first detection means (50; 51), which are able to detect the fluorescence emitted by the spermatozoa (SP) in a direction substantially perpendicular to the principal plane of symmetry (P1) of the transport channel, and more particularly at least in a direction perpendicular to the main plane of symmetry (P1) of the transport channel,
(b) said device comprising means for transporting the spermatozoa (SP) one behind the other in the transport channel (100) with substantially the same spatial orientation of each spermatozoon, the fluorescence detection means (5) comprising at least first detection means (50 ; 51) which are oriented in the direction of one of the two main faces (F) having the largest surface of each spermatozoon (SP) successively passing in the transport channel (100) in front of said detection means and being spatially-oriented, and the device being **characterized in that** the electronic processing means (7) are able to automatically discriminate the spermatozoa (SP) using at least the duration (D) of each fluorescence signature (PEAK) that is characteristic of a spermatozoon in the fluorescence detection signal (50a) generated by the first detection means (50; 51).

2. Device according to Claim 1, in which the electronic processing means (7) are able to automatically measure the duration (D) of each fluorescence signature (PEAK) above a predefined fluorescence threshold (Sₘᵢₙ) and to automatically discriminate spermatozoa (SP) using at least said duration (D), and preferably wherein the electronic processing means (7) are able to automatically detect in the fluorescence detection signal (50a) generated by the first detection means (50; 51) the point in time (t0) where the fluorescence detection signal (50a) passes above the predefined fluorescence threshold (Sₘᵢₙ) and the point in time (t1) where the fluorescence detection signal (50a) returns below the predefined fluorescence threshold (Sₘᵢₙ), and are able to measure said duration (D= t1 - t0) of the fluorescence signature (PEAK) as being the time interval between these two detected point in time.

3. Device according to any preceding claim, wherein the fluorescence detecting means further includes second detecting means (52; 53) adapted to detect the fluorescence emitted by the spermatozoa (SP), and wherein the electronic processing means (7) are able to automatically discriminate the spermatozoa (SP) circulating in the transport channel, from the fluorescence detected by the second detection means (52; 53), and more particularly from the maximum intensity maximum (MAX) of the fluorescence detected by the second detection means (52; 53).

4. Device according to claim 3, wherein the electronic processing means (7) are able to automatically discriminate the spermatozoa (SP) circulating in the transport channel at least from the duration (D) of each fluorescence signature (PEAK) that characterize spermatozoa in the fluorescence detection signal (50a) generated by the first detection means (50; 51) and at least when the maximum intensity (MAX) of the fluorescence detected by the second detection means (52; 53) is below a predefined maximum threshold (Iₘₐₓ).

5. Device according to claim 4, wherein the second detection means (52; 53) are able to detect the fluorescence emitted by the spermatozoa (SP) in a direction substantially perpendicular to the secondary plane (P2) of the transport channel which is perpendicular to the main plane of symmetry (P1), and more particularly at least in a direction perpendicular to the secondary plane (P2) of the transport channel.

6. Device according to any preceding claim, comprising means (2) for injecting a solution (S) containing spermatozoa (SP) labeled by means of at least one fluorochrome into said transport channel (100).

7. Device according to any one of the preceding claims, comprising hydrodynamic focusing means (3; 101) for injecting a liquid (L) into said transport channel (100) so as to induce the spermatozoa (SP) in the transport channel (100), positioning them substantially in a hydrodynamic focusing plane and spacing them one behind the other and preferably wherein the hydrodynamic focusing means (3; 101), in combination with the transport channel (100), make it possible to spatially orient the spermatozoa transported in the transport channel with the same spatial orientation.

8. Device according to any preceding claim, wherein the means of excitation (4') are able to locally illuminate the labeled sperm (SP) circulating in said transport channel (100) by means of electromagnetic fluorescence excitation radiation (REF) that propagates at least in a direction substantially parallel to the main plane of symmetry (P1) of the transport channel.

9. Device according to any one of the preceding claims, in which the electronic processing means allow the discrimination between a first and second type of spermatozoa and in particular the discrimination between X spermatozoa and Y spermatozoa, the device comprises means of selection (6) for selectively altering the spermatozoa (SP) of one of the two types that circulate in the transport channel (100), or comprises sorting means for separating the first type of spermatozoa (SP) from the second type of spermatozoa (SP).

10. Device according to Claim 9, in which the means of selection (6) comprise a source of electromagnetic radiation (60) which is able to be controlled automatically by the processing means (7) so as to selectively alter the spermatozoa (SP) circulating in the transport channel (100), by means of the electromagnetic radiation (R) emitted by the source of electromagnetic radiation (60).

11. Device according to any one of the preceding claims, wherein the transport channel (100) is a microfluidic channel of which at least one cross-sectional dimension is less than 1 mm, and more particularly less than 100 µm and/or wherein the transport channel (100) has a rectangular cross section.

12. Device according to claim 11, wherein the width (E) of the rectangular transport channel (100) is less than 1 mm, and preferably less than 100 µm.

13. Use of the device according to any one of the preceding claims to automatically discriminate, and where appropriate to automatically select or automatically sort spermatozoa *in vitro.*

14. *In vitro* sperm (SP) discrimination procedure, in which a solution (S) containing the spermatozoa (SP) labeled by means of at least one fluorochrome is injected into the transport channel (100) of the device of any one of claims 1 to 12, said spermatozoa (SP) are circulated in the transport channel (100) one behind the other by spatially orienting them with the same spatial orientation, and in which the chromosome type of each spermatozoon circulating in the transport channel (100) is automatically detected using at least the duration (D) of the fluorescence signature (PEAK) that characterizes a spermatozoon in the fluorescence detection signal (50a) delivered by the first detection means (50; 51) of the device.

15. A process for sexed semen production by *in vitro* selecting or sorting of spermatozoa (SP) by means of the device of any one of claims 1 to 12, during which a solution (S) containing the spermatozoa (SP) labeled with at least one fluorochrome is injected in the transport channel (100) of the device, said spermatozoa (SP) are circulated in the transport channel (100) one behind the other, orienting them spatially with the same spatial orientation, the chromosome type of each spermatozoon circulating in the transport channel (100) is automatically detected, and in particular, if the spermatozoon is of type X or Y, using at least the duration (D) of the fluorescence signature (PEAK) that characterizes a spermatozoon in the fluorescence detection signal (50a) delivered by the first detecting means (50; 51) of the device, and the selection of a given type of spermatozoa (SP), in particular by altering the other type of spermatozoa (SP), or spermatozoa (SP) are sorted according to their type, and in particular their X or Y type, in particular by separating spermatozoa (SP) of a given type from spermatozoa (SP) of the other type.
